(19) Europäisches Patentamt / European Patent Office / Office européen des brevets

(11) **EP 4 101 832 B1**

(12) **EUROPEAN PATENT SPECIFICATION**

(45) Date of publication and mention of the grant of the patent:
**15.11.2023 Bulletin 2023/46**

(21) Application number: **22177601.6**

(22) Date of filing: **07.06.2022**

(51) International Patent Classification (IPC):
***C07C 29/147*** *(2006.01)* ***C07C 33/12*** *(2006.01)*
***C07C 51/09*** *(2006.01)* ***C07C 57/26*** *(2006.01)*
***C07C 67/00*** *(2006.01)* ***C07C 67/08*** *(2006.01)*
***C07C 69/145*** *(2006.01)* ***C07C 69/608*** *(2006.01)*

(52) Cooperative Patent Classification (CPC):
(C-Sets available)
**C07C 67/00; C07C 29/147; C07C 51/09; C07C 67/08;** C07C 2601/10 (Cont.)

(54) **A PROCESS FOR PREPARING 2-(1,5,5-TRIMETHYL-2-CYCLOPENTENYL)ETHYL ACETATE**

VERFAHREN ZUR HERSTELLUNG VON 2-(1,5,5-TRIMETHYL-2-CYCLOPENTENYL)-ETHYLACETAT

PROCÉDÉ DE PRÉPARATION DE L'ACÉTATE DE 2-(1,5,5-TRIMÉTHYL-2-CYCLOPENTÉNYLE) ÉTHYLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2021 JP 2021096730**

(43) Date of publication of application:
**14.12.2022 Bulletin 2022/50**

(73) Proprietor: **Shin-Etsu Chemical Co., Ltd.**
**Tokyo 100-0005 (JP)**

(72) Inventors:
- **YAMASHITA, Miyoshi**
  **Niigata, 942-8601 (JP)**
- **KINSHO, Takeshi**
  **Niigata, 942-8601 (JP)**

(74) Representative: **De Vries & Metman**
**Overschiestraat 180**
**1062 XK Amsterdam (NL)**

(56) References cited:
- **REMYA RAMESH ET AL: "Enantiospecific Synthesis of Both Enantiomers of the Longtailed Mealybug Pheromone and Their Evaluation in a New Zealand Vineyard", THE JOURNAL OF ORGANIC CHEMISTRY, vol. 80, no. 15, 7 August 2015 (2015-08-07), pages 7785-7789, XP055306009, ISSN: 0022-3263, DOI: 10.1021/acs.joc.5b01131**
- **J.G. MILLAR ET AL: "Improved synthesis of the pheromone of the long-tailed mealybug", SYNLETT, GEORG THIEME VERLAG, DE, no. 15, 30 August 2010 (2010-08-30), pages 2319-2321, XP002721999, ISSN: 0936-5214, DOI: 10.1055/S-0030-1258025 [retrieved on 2010-08-30]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 29/147, C07C 33/12;**
**C07C 51/09, C07C 57/26;**
**C07C 67/00, C07C 69/608;**
**C07C 67/08, C07C 69/145**

## Description

TECHNICAL FIELD

The present invention relates to a process for preparing 2-(1,5,5-trimethyl-2-cyclopentenyl)ethyl acetate.

BACKGROUND ART

Longtailed mealybug (scientific name: *Pseudococcus longispinus)* is a pest belonging to the order *Hemiptera,* the family *Pseudococcidae,* and suck juice of many agricultural plants, such as grapes, apples, citrus, and pears to damage these agricultural plants. Further, longtailed mealybugs discharge honeydew which contains sugar to cause fungi-induced diseases. These damage and diseases reduce the yield and quality of such agricultural plants, which is a serious problem.

Generally, insecticides have been used for controlling mealybugs. However, mealybugs live in narrow spaces in leaves and/or plant barks and are themselves covered with a waxy substance. Therefore, insecticides are difficult to come in contact with the pest body. This results in insufficient effects of the insecticides.

In the light of adverse effects of insecticides on the environment and human health, there is recently a demand for the development of a new highly safe, and eco-friendly control method such as mating disruption and/or mass trapping using sex pheromones of insects. Development of such a new control method requires the industrial and inexpensive preparation of a sex pheromone in a large amount.

A sex pheromone of the longtailed mealybug is reported to be an optically active (-)-2-(1,5,5-trimethyl-2-cyclopentenyl)ethyl acetate (Non-Patent Literature 1 listed below). Further, it is reported that there is no difference in the attracting activity for the longtailed mealybug between (-)-2-(1,5,5-trimethyl-2-cyclopentenyl)ethyl acetate which is this sex pheromone and a racemic (±)-2-(1,5,5-trimethyl-2-cyclopentenyl)ethyl acetate which is an equivalent mixture of the enantiomers (Non-Patent Literature 1). Therefore, for the establishment of a technique for controlling the pests utilizing sex pheromones, it is thought to be efficient to find a process for preparing the racemic (±)-2-(1,5,5-trimethyl-2-cyclopentenyl)ethyl acetate, in view of the inexpensive supply of the pheromone and for an economical control method.

One process for preparing this racemic pheromone is reported as follows. 3,4,4-Trimethyl-2-cyclopenten-1-ol is acetylated, and then, the reaction product is reacted with a base, and subsequently, reacted with t-butyldimethylsilyl chloride, the reaction product is subjected to an Ireland-Claisen rearrangement reaction to form (1,5,5-trimethyl-2-cyclopentenyl)acetic acid as an intermediate, and then, the functional group of the intermediate is converted into the functional group of the target compound (Non-Patent Literature 2 listed below).

Like the aforesaid Ireland-Claisen rearrangement reaction, a Johnson-Claisen rearrangement reaction is known as a Claisen-type rearrangement reaction of allylalcohols such as a 2-cyclopenten-1-ol compound, in which the 2-cyclopenten-1-ol compound is reacted with a trialkyl orthoacetate in the presence of a weakly acidic catalyst such as propionic acid to form a (2-cyclopentenyl)acetate compound (Non-Patent Literature 3 listed below).

LIST OF THE LITERATURES

[Non-Patent Literatures]

[Non-Patent Literature 1] R. Ramesh et al., J. Org. Chem., 2015, 80, 7785-7789.
[Non-Patent Literature 2] J. G. Millar et al., Synlett, 15 (2010) 2319-2321.
[Non-Patent Literature 3] W. S. Johnson et al., J. Am. Chem. Soc., 92 (1970), 92, 741-743.


PROBLEMS TO BE SOLVED BY THE INVENTION

However, the preparation process described in Non-Patent Literature 2 using an Ireland-Claisen rearrangement reaction has such disadvantages that an organolithium compound and lithium amide, which are ignitable, are used; the reaction must be carried out at an extremely low temperature such as -78°C; and industrially relatively expensive trialkylsilane chloride is used.

In the process described in Non-Patent Literature 3 for preparing a (2-cyclopentenyl)acetate compound by a Johnson-Claisen rearrangement reaction in which a 2-cyclopenten-1-ol compound is reacted with a trialkyl orthoacetate in the presence of a weakly acidic catalyst, a dehydration reaction of the starting material, 2-cyclopenten-1-ol compound, occurs preferentially to cause a problematic low yield (see Non-Patent Literature 2 and the Comparative example 1 described in the present specification). Thus, the prior art fails to industrially and economically prepare a (1,5,5-trimethyl-2-cyclopentenyl)acetic acid and a (1,5,5-trimethyl-2-cyclopentenyl)acetate compound as the intermediate in a large amount.

SUMMARY OF THE INVENTION

The present invention has been made in these circumstances, and aims to overcome the aforesaid problems of the prior art and to provide an industrial and economical process for preparing 2-(1,5,5-trimethyl-2-cyclopentenyl)ethyl acetate.

As a result of intensive research, the present inventors have now provided a haloacetaldehyde alkyl 3,4,4-trimethyl-2-cyclopentenyl acetal compound which is a novel compound, and have found that it is possible to prepare a (1,5,5-trimethyl-2-cyclopentenyl)acetate compound, without using an ignitable starting material and an industrially expensive starting material, in an industrially readily applicable range of a reaction temperature, by subjecting the haloacetaldehyde alkyl 3,4,4-trimethyl-2-cyclopentenyl acetal compound to a dehydrohalogenation reaction in the presence of a base, followed by a rearrangement reaction.

The present inventors have also found that it is possible to efficiently and industrially prepare 2-(1,5,5-trimethyl-2-cyclopentenyl)ethyl acetate by subjecting the alkoxycarbonylmethyl group (i.e., $-CH_2C(=O)OR$) of the (1,5,5-trimethyl-2-cyclopentenyl)acetate compound thus prepared to a multi-step conversion to be converted into a 2-acetoxyethyl group (i.e., $-CH_2CH_2OAc$) and, thus, have completed the present invention.

According to one aspect of the present invention, the present invention provides a process for preparing 2-(1,5,5-trimethyl-2-cyclopentenyl)ethyl acetate (3) of the following formula (3):

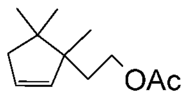

(3)

wherein Ac represents an acetyl group,
the process comprising:
subjecting a haloacetaldehyde alkyl 3,4,4-trimethyl-2-cyclopentenyl acetal compound of the following general formula (1):

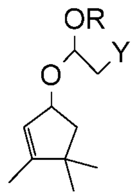

(1)

wherein R represents a linear or branched alkyl group having 1 to 4 carbon atoms, and Y represents a halogen atom, to a dehydrohalogenation reaction in the presence of a base, followed by a rearrangement reaction to obtain a (1,5,5-trimethyl-2-cyclopentenyl)acetate compound of the following general formula (2):

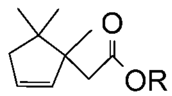

(2)

wherein R is as defined above, and
subjecting the alkoxycarbonylmethyl group (i.e., $-CH_2C(=O)OR$) of the (1,5,5-trimethyl-2-cyclopentenyl)acetate compound (2) to a multi-step conversion to be converted into a 2-acetoxyethyl group (i.e., $-CH_2CH_2OAc$) to obtain 2-(1,5,5-trimethyl-2-cyclopentenyl)ethyl acetate (3).

According to the present invention, the (1,5,5-trimethyl-2-cyclopentenyl)acetate compound (2) may be prepared without an ignitable starting material and an industrially expensive starting material, in an industrially readily applicable range of a reaction temperature. 2-(1,5,5-Trimethyl-2-cyclopentenyl)ethyl acetate (3) may be industrially and economically prepared by subjecting an alkoxycarbonylmethyl group (i.e., - $CH_2C(=O)OR$) of the (1,5,5-trimethyl-2-cyclopentenyl)acetate compound (2) thus obtained to a multi-step conversion to be converted into a 2-acetoxyethyl group (i.e., -

$CH_2CH_2OAc$).

DETAILED DESCRIPTION OF THE PREFERRED EMBODIMENTS

Embodiments of the present invention will be described in detail below. It should be noted that the present invention is not limited to or by the embodiments.

A. Haloacetaldehyde alkyl 3,4,4-trimethyl-2-cyclopentenyl acetal compound which is a novel compound and of the following general formula (1) will be described below.

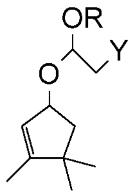

(1)

In the general formula (1), R represents a linear or branched alkyl group having 1 to 4 carbon atoms, and Y represents a halogen atom.

Examples of the alkyl group include linear alkyl groups such as a methyl group, an ethyl group, an n-propyl group, and an n-butyl group; and branched alkyl groups such as an isopropyl group and an isobutyl group. A methyl group, an ethyl group, and an n-propyl group are preferred in view of the reactivity and/or the yield.

Examples of the halogen atom include a chlorine atom, a bromine atom, and an iodine atom. A bromine atom or an iodine atom is preferred in view of the reactivity and/or the yield.

Examples of the haloacetaldehyde alkyl 3,4,4-trimethyl-2-cyclopentenyl acetal compound (1) include the following compounds:

chloroacetaldehyde linear alkyl 3,4,4-trimethyl-2-cyclopentenyl acetal compounds such as chloroacetaldehyde methyl 3,4,4-trimethyl-2-cyclopentenyl acetal, chloroacetaldehyde ethyl 3,4,4-trimethyl-2-cyclopentenyl acetal, chloroacetaldehyde n-propyl 3,4,4-trimethyl-2-cyclopentenyl acetal, and chloroacetaldehyde n-butyl 3,4,4-trimethyl-2-cyclopentenyl acetal;
bromoacetaldehyde linear alkyl 3,4,4-trimethyl-2-cyclopentenyl acetal compounds such as bromoacetaldehyde methyl 3,4,4-trimethyl-2-cyclopentenyl acetal, bromoacetaldehyde ethyl 3,4,4-trimethyl-2-cyclopentenyl acetal, bromoacetaldehyde n-propyl 3,4,4-trimethyl-2-cyclopentenyl acetal, and bromoacetaldehyde n-butyl 3,4,4-trimethyl-2-cyclopentenyl acetal;
iodoacetaldehyde linear alkyl 3,4,4-trimethyl-2-cyclopentenyl acetal compounds such as iodoacetaldehyde methyl 3,4,4-trimethyl-2-cyclopentenyl acetal, iodoacetaldehyde ethyl 3,4,4-trimethyl-2-cyclopentenyl acetal, iodoacetaldehyde n-propyl 3,4,4-trimethyl-2-cyclopentenyl acetal, and iodoacetaldehyde n-butyl 3,4,4-trimethyl-2-cyclopentenyl acetal;
chloroacetaldehyde branched alkyl 3,4,4-trimethyl-2-cyclopentenyl acetal compounds such as chloroacetaldehyde isopropyl 3,4,4-trimethyl-2-cyclopentenyl acetal, and chloroacetaldehyde isobutyl 3,4,4-trimethyl-2-cyclopentenyl acetal;
bromoacetaldehyde branched alkyl 3,4,4-trimethyl-2-cyclopentenyl acetal compounds such as bromoacetaldehyde isopropyl 3,4,4-trimethyl-2-cyclopentenyl acetal, and bromoacetaldehyde isobutyl 3,4,4-trimethyl-2-cyclopentenyl acetal; and
iodoacetaldehyde branched alkyl 3,4,4-trimethyl-2-cyclopentenyl acetal compounds such as iodoacetaldehyde isopropyl 3,4,4-trimethyl-2-cyclopentenyl acetal, and iodoacetaldehyde isobutyl 3,4,4-trimethyl-2-cyclopentenyl acetal.

Specifically, the bromoacetaldehyde linear alkyl 3,4,4-trimethyl-2-cyclopentenyl acetal compounds and the iodoacetaldehyde linear alkyl 3,4,4-trimethyl-2-cyclopentenyl acetal compounds are preferred as the haloacetaldehyde alkyl 3,4,4-trimethyl-2-cyclopentenyl acetal compound (1) in view of the reactivity.

Furthermore, the haloacetaldehyde alkyl 3,4,4-trimethyl-2-cyclopentenyl acetal compound (1) may be its enantiomers, diastereomers, and a mixture of such stereoisomers in the same or different amounts.

Next, a process for preparing the haloacetaldehyde alkyl 3,4,4-trimethyl-2-cyclopentenyl acetal compound (1) will be described below.

The haloacetaldehyde alkyl 3,4,4-trimethyl-2-cyclopentenyl acetal compound (1) may be prepared, for example, by halogenating an alkyl vinyl ether compound of the following general formula (7) with a halogenating agent to form a

halide and, subsequently, subjecting the halide thus obtained to a substitution reaction with 3,4,4-trimethyl-2-cyclopenten-1-ol of the following formula (8), as shown in the following reaction formula.

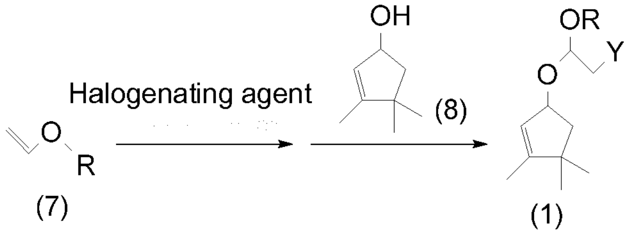


(7) (8) (1)

The process for preparing the haloacetaldehyde alkyl 3,4,4-trimethyl-2-cyclopentenyl acetal compound (1) will be further described in detail below.

The alkyl vinyl ether compound (7), which is the starting material, will be described below.

R in the general formula (7) is as defined for the general formula (1).

Examples of the alkyl vinyl ether compound (7) include linear alkyl vinyl ethers such as methyl vinyl ether, ethyl vinyl ether, n-propyl vinyl ether, and n-butyl vinyl ether, and branched alkyl vinyl ethers such as isopropyl vinyl ether and isobutyl vinyl ether.

The alkyl vinyl ether compound (7) may be commercially available one or may be prepared in house.

The halogenation of the alkyl vinyl ether compound (7) may be carried out with a halogenating agent and may be carried out with heating or cooling, if needed.

Examples of the halogenating agent used in the halogenation include chlorinating agents such as chlorine, sulfuryl chloride, N-chlorosuccinimide, iodobenzene dicholoride, tetrabutylammonium iodotetrachloride, titanium (IV) chloride, and copper (II) chloride; brominating agents such as bromine, N-bromosuccinimide, N-bromoacetamide, 1,3-dibromo-5,5-dimethylhydantoin, tetrabutylammonium tribromide, phenyltrimethylammonium tribromide, iodobenzene dibromide, copper (II) bromide, copper (I) bromide, magnesium (II) bromide, and aluminum bromide; iodinating agents such as iodine, N-iodosuccinimide, and 1,3-diiodo-5,5-dimethylhydantoin; and chloro-iodinating agents such as iodine monochloride and potassium tetrachloroiodate. The brominating agent and the iodinating agent are preferred. Bromine and N-bromosuccinimide among the brominating agent, and iodine and N-iodosuccinimide among the iodinating agents are more preferred in view of the reactivity and/or the yield.

An amount of the halogenating agent used in the halogenation varies, depending on the structure of the reducing agent and/or the reactivity of the alkyl vinyl ether compound (7) and/or the halogenating agent, and is preferably from 0.2 mol to 5.0 mol, more preferably from 0.5 mol to 2.0 mol, per mol of the alkyl vinyl ether compound (7) in view of the yield and/or the by-production of an impurity.

A solvent used in the halogenation may be any solvent that has no adverse effect on the halogenation. Examples of the solvent used in the halogenation include halogen-based solvents such as methylene chloride, chloroform, carbon tetrachloride, and 1,2-dichloroethane; ether solvents such as diethyl ether, di-n-butyl ether, di-t-butyl ether, tetrahydrofuran, 1,4-dioxane, and diethyleneglycol dimethyl ether; hydrocarbon solvents such as hexane and heptane; aprotic polar solvents such as N,N-dimethylformamide, N,N-dimethylacetamide, dimethyl sulfoxide, and hexamethylphosphoric triamide. Halogen-based solvents, ether solvents, and aprotic polar solvents are preferred in view of the reactivity.

The solvent may be used either alone or in combination thereof, if necessary, and may be optionally determined while considering the type and/or the reactivity and/or the selectivity of the alkyl vinyl ether compound (7) and/or the halogenating agent.

An amount of the solvent used in the halogenation may be optionally determined while considering the type and/or the reactivity of the alkyl vinyl ether compound (7) and/or halogenating agent, and is for example, preferably from 50 g to 10,000 g, more preferably from 500 g to 8,000 g, per mol of the alkyl vinyl ether compound (7) in view of the reactivity and/or the by-production of an impurity.

A reaction temperature of the halogenation may be optionally determined while considering the reactivity of the alkyl vinyl ether compound (7) and/or the halogenating agent and/or the formation of an impurity, and is, for example, preferably from -60°C to 150°C, more preferably from -20°C to 50°C, in view of the reactivity and/or the formation of an impurity.

The reaction time of the halogenation is preferably optimized, depending on the reactivity of the alkyl vinyl ether compound (7) and/or the halogenating agent, by monitoring the reaction progress with, for example, gas chromatography and/or thin layer chromatography to confirm the disappearance of the alkyl vinyl ether compound (7) and/or the halogenating agent. For example, the reaction time of the halogenation is preferably from 0.5 hours to 168 hours, more preferably from 0.5 hours to 24 hours, even more preferably from 0.5 hours to 6 hours, in view of the yield and/or the formation of an impurity.

A halide prepared from the halogenation of the alkyl vinyl ether compound (7) with the halogenating agent is thought to be an alkyl 1,2-dihaloethyl ether of the following general formula (9). The halide may be isolated and/or purified after the halogenation, and then used in a subsequent step or may be used as such in the reaction mixture in a subsequent step without isolation and/or purification after the halogenation.

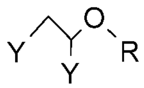

(9)

In the general formula (9), R is as defined for the general formula (7), and Y represents, independently of each other, a halogen atom. Examples of the halogen atom include a chlorine atom, a bromine atom, and an iodine atom. A bromine atom or an iodine atom is preferred in view of the yield and/or the reactivity. Y may be, independently of each other, the same or different. When the halogenating agent is, for example, a chloro-iodinating agent such as iodine monochloride or potassium tetrachloroiodate, Y may be, independently of each other, different.

Next, the 3,4,4-trimethyl-2-cyclopenten-1-ol (8) may be commercially available one or may be prepared in house.

In the substitution reaction, the halogen atom (i.e., the secondary Y) in the halide is substituted with the 3,4,4-trimethyl-2-cyclopenten-1-ol (8) to form the haloacetaldehyde alkyl 3,4,4-trimethyl-2-cyclopentenyl acetal compound (1).

An amount of the 3,4,4-trimethyl-2-cyclopenten-1-ol (8) used is preferably from 0.2 mol to 5.0 mol, more preferably from 0.5 mol to 2.0 mol, per mol of the alkyl vinyl ether compound (7) in view of the yield and/or the formation of a by-product and/or the economy.

The substitution reaction may be carried out with heating or cooling, if needed. The substitution reaction may be carried out in the presence of a base while considering the reactivity of the 3,4,4-trimethyl-2-cyclopenten-1-ol (8) and the halide and/or the by-production of an impurity.

Examples of the base include amines such as triethylamine, diisopropylethylamine, pyridine, N,N-dimethylaminopyridine, and N,N-dimethylaniline; organometallic compounds such as n-butyl lithium, methyl lithium, and phenyl lithium; metal amides such as lithium diisopropylamide, lithium hexamethyldisilazide, sodium hexamethyldisilazide, and lithium dicyclohexylamide; metal hydroxides such as sodium hydroxide and potassium hydroxide; and metal carbonates such as potassium carbonate, sodium carbonate, and sodium bicarbonate.

The base may be used either alone or in combination thereof, if necessary, and may be optionally determined while considering the reactivity of the 3,4,4-trimethyl-2-cyclopenten-1-ol (8) and/or the halide and/or the formation of an impurity.

An amount of the base used may be optionally determined while considering the reactivity of the 3,4,4-trimethyl-2-cyclopenten-1-ol (8) and/or the halide and/or the formation of an impurity, and is, for example, preferably from 0.5 mol to 5.0 mol, more preferably from 0.8 mol to 2.0 mol, per mol of the 3,4,4-trimethyl-2-cyclopenten-1-ol (8) in view of the reactivity and/or economy.

A solvent used in the substitution reaction may be any solvent that has no adverse effect on the substitution reaction. Examples of the solvent used in the substitution reaction include halogen-based solvents such as methylene chloride, chloroform, carbon tetrachloride, and 1,2-dichloroethane; hydrocarbon solvents such as hexane, heptane, benzene, and toluene; ether solvents such as diethyl ether, tetrahydrofuran, 1,4-dioxane, and diethylene glycol dimethyl ether; nitrile solvents such as acetonitrile; ketone solvents such as acetone, methyl ethyl ketone, and diisobutyl ketone; ester solvents such as ethyl acetate and butyl acetate; and aprotic polar solvents such as N,N-dimethylformamide, N,N-dimethylacetamide, dimethyl sulfoxide, and hexamethylphosphoric triamide. The halogen-based solvents, the ether solvents, and the aprotic polar solvents are preferred in view of the reactivity and/or the yield.

The solvent may be used either alone or in combination thereof, if necessary, and may be optionally determined while considering the type and/or the reactivity and/or the selectivity of the 3,4,4-trimethyl-2-cyclopenten-1-ol (8) and/or the halide.

A solvent used in the substitution reaction may be the solvent already used in the halogenation as such. The same species of solvent as in the halogenation or any solvent different from the solvent used in the halogenation may be added into the substitution reaction system to increase the reactivity and/or adjust the concentration.

An amount of the solvent used in the substitution reaction may be optionally determined while considering the reactivity of the 3,4,4-trimethyl-2-cyclopenten-1-ol (8) and/or the halide and/or the formation of an impurity, and is, for example, preferably from 50 g to 10,000 g, more preferably from 500 g to 8,000 g, per mol of the 3,4,4-trimethyl-2-cyclopenten-1-ol (8) in view of the reactivity and/or the by-production of an impurity.

A reaction temperature of the substitution reaction may be optionally determined while considering the reactivity of the 3,4,4-trimethyl-2-cyclopenten-1-ol (8) and/or the halide and/or the formation of an impurity and is, for example, preferably from -60°C to 150°C, more preferably from -20°C to 50°C, in view of the reactivity and/or the formation of an impurity.

The reaction time of the substitution reaction is preferably optimized, depending on the reactivity of the 3,4,4-trimethyl-2-cyclopenten-1-ol (8) and/or the halide, by monitoring the reaction progress with, for example, gas chromatography and/or thin layer chromatography to confirm the disappearance of the 3,4,4-trimethyl-2-cyclopenten-1-ol (8) and/or the halide. For example, the reaction time of the substitution reaction is preferably from 1 hour to 168 hours, more preferably from 1 hour to 24 hours, even more preferably from 1 hour to 6 hours, in view of the yield and/or the formation of an impurity.

The haloacetaldehyde alkyl 3,4,4-trimethyl-2-cyclopentenyl acetal compound (1) formed in the substitution reaction may be suitably isolated and/or purified in any purification method used in usual organic synthesis such as distillation at a reduced pressure and/or various chromatography. Distillation at a reduced pressure is preferred in view of the industrial economy. When the haloacetaldehyde alkyl 3,4,4-trimethyl-2-cyclopentenyl acetal compound (1) formed in the substitution reaction has a sufficient purity, the crude product comprising the haloacetaldehyde alkyl 3,4,4-trimethyl-2-cyclopentenyl acetal compound (1) may be used as such without purification in a subsequent step.

B. Next, a process for preparing the (1,5,5-trimethyl-2-cyclopentenyl)acetate compound of the following general formula (2) will be described below.

The (1,5,5-trimethyl-2-cyclopentenyl)acetate compound (2) is prepared by subjecting the haloacetaldehyde alkyl 3,4,4-trimethyl-2-cyclopentenyl acetal compound (1) to a dehydrohalogenation (-HY) reaction in the presence of a base, followed by a rearrangement reaction, as shown in the following reaction formula (see Example 1-1 to Example 1-6 below).

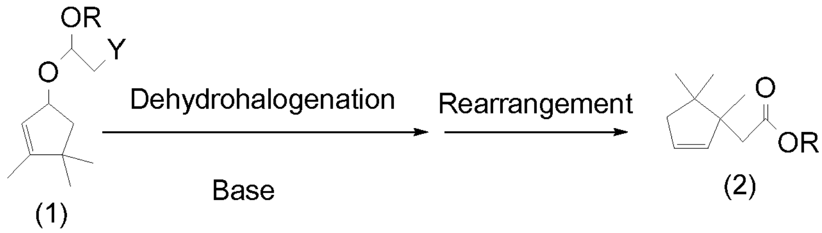


The haloacetaldehyde alkyl 3,4,4-trimethyl-2-cyclopentenyl acetal compound (1), which is the starting material, is as mentioned above. R in the general formula (1) is as defined above and is preferably a linear alkyl group having 1 to 4 carbon atoms in view of the reactivity. Linear alkyl groups having 1 to 3 carbon atoms, which are a methyl group, an ethyl group, and an n-propyl group, are more preferred in view of the reactivity and/or the availability.

Next, the (1,5,5-trimethyl-2-cyclopentenyl)acetate compound (2) to be formed in the rearrangement reaction will be described below.

R in the general formula (2) is as defined for the general formula (1).

Examples of the (1,5,5-trimethyl-2-cyclopentenyl)acetate compound (2) include linear alkyl (1,5,5-trimethyl-2-cyclopentenyl)acetate compounds such as methyl(1,5,5-trimethyl-2-cyclopentenyl)acetate, ethyl(1,5,5-trimethyl-2-cyclopentenyl)acetate, n-propyl(1,5,5-trimethyl-2-cyclopentenyl)acetate, and n-butyl(1,5,5-trimethyl-2-cyclopentenyl)acetate; and branched alkyl (1,5,5-trimethyl-2-cyclopentenyl)acetate compounds such as isopropyl(1,5,5-trimethyl-2-cyclopentenyl)acetate, and isobutyl(1,5,5-trimethyl-2-cyclopentenyl)acetate.

Furthermore, when R in the general formula (2) does not have any asymmetric carbon atom, the (1,5,5-trimethyl-2-cyclopentenyl)acetate compound (2) includes an (R) (1,5,5-trimethyl-2-cyclopentenyl)acetate compound, an (S) (1,5,5-trimethyl-2-cyclopentenyl)acetate compound, and the racemic and scalemic mixtures thereof. On the other hand, when R in the general formula (2) has one or more asymmetric carbon atoms, the (1,5,5-trimethyl-2-cyclopentenyl)acetate compound (2) may be its enantiomers, diastereomers, and a mixture of such stereoisomers in the same or different amounts.

The dehydrohalogenation reaction may be carried out in the presence of a base, and may be carried out with heating or cooling, if needed.

Examples of the base used in the dehydrohalogenation reaction include metal alkoxides such as sodium methoxide, sodium ethoxide, sodium t-butoxide, lithium methoxide, lithium ethoxide, lithium t-butoxide, potassium methoxide, potassium ethoxide, and potassium t-butoxide; metal hydroxides such as sodium hydroxide, lithium hydroxide, and potassium hydroxide; organometallic reagents such as methyl lithium, ethyl lithium, n-butyl lithium and methylmagnesium chloride; metal amides such as lithium diisopropylamide, lithium hexamethyldisilazide, sodium hexamethyldisilazide, and lithium dicyclohexylamide; and organic nitrogen compounds such as triethylamine, diisopropylethylamine, tributylamine, N,N-dimethylaniline, pyridine, 4-dimethylaminopyridine, pyrrolidine, piperidine, collidine, lutidine, morpholine, piperazine, 1,8-diazabicyclo[5.4.0]undeca-7-ene, and 1,5-diazabicyclo[4.3.0]nona-5-ene. Metal alkoxides are preferred in view of the reactivity and/or the formation of an impurity, and sodium t-butoxide, lithium t-butoxide, and potassium t-butoxide are more preferable.

The base may be used either alone or in combination thereof, if necessary, and may be optionally determined while considering the type and/or the reactivity and/or the selectivity of the haloacetaldehyde alkyl 3,4,4-trimethyl-2-cyclopentenyl acetal compound (1).

An amount of the base used varies, depending on the structure and/or the reactivity of the haloacetaldehyde alkyl 3,4,4-trimethyl-2-cyclopentenyl acetal compound (1). For example, the amount is preferably from 0.2 mol to 5.0 mol, more preferably from 0.5 mol to 2.0 mol, per mol of the haloacetaldehyde alkyl 3,4,4-trimethyl-2-cyclopentenyl acetal compound (1) in view of the yield and/or the formation of an impurity.

A solvent used in the dehydrohalogenation reaction may be any solvent that has no adverse effect on the dehydrohalogenation reaction. Examples of the solvent used in the dehydrohalogenation reaction include alcoholic solvents such as methanol, ethanol, isopropyl alcohol, and t-butyl alcohol; ether solvents such as diethyl ether, di-n-butyl ether, di-t-butyl ether, tetrahydrofuran, 1,4-dioxane, diethyleneglycol dimethyl ether, and diethyleneglycol diethyl ether; hydrocarbon solvents such as hexane, heptane, benzene, toluene, and xylene; aprotic polar solvents such as N,N-dimethylformamide, N,N-dimethylacetamide, 1,3-dimethyl-2-imidazolidinone, dimethyl sulfoxide, and hexamethylphosphoric triamide; and nitrile solvents such as acetonitrile. The ether solvents and the aprotic polar solvents are preferred in view of the reactivity, and N,N-dimethylformamide, N,N-dimethylacetamide, diethyleneglycol dimethyl ether, and diethyleneglycol diethyl ether are more preferred in view of the reactivity.

The solvent may be used either alone or in combination thereof, if necessary, and may be optionally determined while considering the type and/or the reactivity and/or the selectivity of the haloacetaldehyde alkyl 3,4,4-trimethyl-2-cyclopentenyl acetal compound (1).

An amount of the solvent used may be optionally determined while considering the reactivity and/or the solubility of the haloacetaldehyde alkyl 3,4,4-trimethyl-2-cyclopentenyl acetal compound (1) and is, for example, preferably from 30 g to 10,000 g, more preferably from 100 g to 5000 g, per mol of the haloacetaldehyde alkyl 3,4,4-trimethyl-2-cyclopentenyl acetal compound (1) in view of the reactivity and/or the economy.

A reaction temperature of the dehydrohalogenation reaction may be optionally determined while considering the reactivity of the haloacetaldehyde alkyl 3,4,4-trimethyl-2-cyclopentenyl acetal compound (1) and/or the formation of an impurity and is, for example, preferably from -60°C to 150°C, more preferably from -20°C to 80°C, in view of the reactivity and/or the formation of an impurity.

The reaction time of the dehydrohalogenation reaction is preferably optimized, depending on the reactivity of the haloacetaldehyde alkyl 3,4,4-trimethyl-2-cyclopentenyl acetal compound (1), by monitoring the reaction progress with, for example, gas chromatography and/or thin layer chromatography to confirm the disappearance of the haloacetaldehyde alkyl 3,4,4-trimethyl-2-cyclopentenyl acetal compound (1) which is a substrate. For example, the reaction time of the dehydrohalogenation reaction is preferably from 1 hour to 168 hours, more preferably from 1 hour to 24 hours, even more preferably from 1 hour to 12 hours, in view of the yield and/or the formation of an impurity.

During the dehydrohalogenation reaction, an alkyl 3,4,4-trimethyl-2-cyclopentenyl ketene acetal compound of the following general formula (4) is thought to be formed in the reaction system as a product of the dehydrohalogenation reaction.

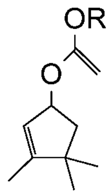

(4)

R in the general formula (4) is as defined for the general formula (1).

The product of the dehydrohalogenation reaction may be isolated and/or purified in any purification method used in usual organic synthesis such as distillation at a reduced pressure and/or various chromatography, and then may be used in a subsequent rearrangement reaction. When the product of the dehydrohalogenation reaction is difficult to be isolated and/or purified due to its nature, the product is preferably used as is in a subsequent rearrangement reaction.

Next, in the rearrangement reaction, a (1,5,5-trimethyl-2-cyclopentenyl)acetate compound (2) is prepared from the product of the dehydrohalogenation reaction via a [3,3]-sigmatropic rearrangement as shown in the following reaction formula. The carbon atom attached to the alkoxycarbonylmethyl group becomes quaternary, resulting in greater steric hindrance of the (1,5,5-trimethyl-2-cyclopentenyl)acetate compound (2) and difficulty in preparation of the (1,5,5-trimethyl-2-cyclopentenyl)acetate compound (2) using usual anion species. Therefore, the preparation method utilizing the [3,3]-sigmatropic rearrangement is thought to be effective in the process for preparing the (1,5,5-trimethyl-2-cyclopen-

tenyl)acetate compound (2).

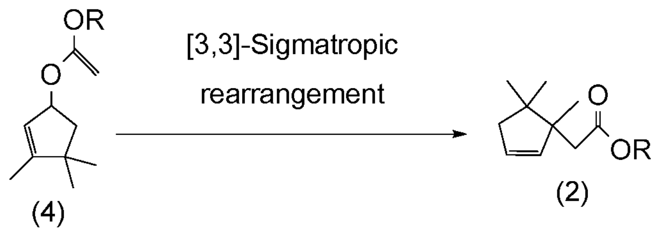


(4) (2)

The rearrangement reaction may be carried out in a solvent or without a solvent, and may be carried out with heating or cooling, if needed.

A solvent used in the rearrangement reaction may be any solvent that has no adverse effect on the rearrangement reaction. Examples of the solvent used in the rearrangement reaction include alcoholic solvents such as methanol, ethanol, isopropyl alcohol, and t-butyl alcohol; ether solvents such as diethyl ether, di-n-butyl ether, di-t-butyl ether, tetrahydrofuran, 1,4-dioxane, and diethyleneglycol dimethyl ether; hydrocarbon solvents such as hexane, heptane, benzene, toluene, and xylene; aprotic polar solvents such as N,N-dimethylformamide, N,N-dimethylacetamide, 1,3-dimethyl-2-imidazolidinone, dimethyl sulfoxide, and hexamethylphosphoric triamide; and nitrile solvents such as acetonitrile. The ether solvents and the aprotic polar solvents are preferred in view of the reactivity.

The solvent may be used either alone or in combination thereof, if necessary, and may be optionally determined while considering the type and/or the reactivity of the product of the dehydrohalogenation reaction.

When the product of the dehydrohalogenation reaction is used in a subsequent rearrangement reaction without being isolated and/or purified, a solvent to be used in the rearrangement reaction may be the solvent itself used in the dehydrohalogenation reaction. Any solvent may be additionally used in the rearrangement reaction system to adjust the reaction temperature and/or concentration.

An amount of the solvent used in the rearrangement reaction may be optionally determined while considering the reactivity and/or the solubility of the product of the dehydrohalogenation reaction which is the substrate for the rearrangement reaction. For example, an amount of the solvent used is preferably from greater than 0 g to 10,000 g, more preferably from 50 g to 3,000 g, per mol of the haloacetaldehyde alkyl 3,4,4-trimethyl-2-cyclopentenyl acetal compound (1) or the product of the dehydrohalogenation reaction in view of the reactivity and/or the economy.

A reaction temperature of the rearrangement reaction may be optionally determined while considering the reactivity of the product of the dehydrohalogenation reaction and/or the formation of an impurity and is, for example, preferably from -60°C to 250°C, more preferably from 0°C to 150°C, even more preferably from 80°C to 120°C, in view of the reactivity and/or the formation of an impurity.

The reaction time of the rearrangement reaction is preferably optimized, depending on the reactivity of the product of the dehydrohalogenation reaction, by monitoring the reaction progress with, for example, gas chromatography and/or thin layer chromatography to confirm the disappearance of the product of the dehydrohalogenation reaction. For example, the reaction time of the halogenation is preferably from 1 hour to 168 hours, more preferably from 1 hour to 24 hours, even more preferably from 1 hour to 6 hours, in view of the yield and/or the formation of an impurity.

The (1,5,5-trimethyl-2-cyclopentenyl)acetate compound (2) formed in the rearrangement reaction may be suitably isolated and/or purified in any purification method used in usual organic synthesis such as distillation at a reduced pressure and/or various chromatography. Distillation at a reduced pressure is preferred in view of the industrial economy. When the (1,5,5-trimethyl-2-cyclopentenyl)acetate compound (2) formed in the rearrangement reaction has a sufficient purity, the crude product comprising (1,5,5-trimethyl-2-cyclopentenyl)acetate compound (2) may be used as such without purification in a subsequent step.

C. The process for preparing 2-(1,5,5-trimethyl-2-cyclopentenyl)ethyl acetate of the following formula (3) will be described below.

2-(1,5,5-Trimethyl-2-cyclopentenyl)ethyl acetate (3) may be obtained by a multi-step conversion of the alkoxycarbonylmethyl group (i.e., $-CH_2C(=O)OR$) of the (1,5,5-trimethyl-2-cyclopentenyl)acetate compound (2) obtained in B to be converted into a 2-acetoxyethyl group (i.e., $-CH_2CH_2OAc$), as shown in the following reaction formula (see Example 2-1 to Example 2-5 below).

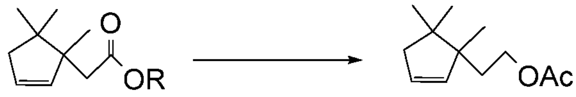

(2) (3)

The (1,5,5-trimethyl-2-cyclopentenyl)acetate compound (2), which is the starting material, is as mentioned above. R in the general formula (2) is as defined for the general formula (1), but is preferably the linear alkyl group having 1 to 4 carbon atoms in view of the reactivity. Linear alkyl groups having 1 to 3 carbon atoms, which are a methyl group, an ethyl group, and an n-propyl group, are more preferred in view of the reactivity and/or the availability.

Next, 2-(1,5,5-trimethyl-2-cyclopentenyl)ethyl acetate (3), which is the target substance, will be described below.

Ac in formula (3) represents an acetyl group.

2-(1,5,5-Trimethyl-2-cyclopentenyl)ethyl acetate (3) includes (R)-2-(1,5,5-trimethyl-2-cyclopentenyl)ethyl acetate, (S)-2-(1,5,5-trimethyl-2-cyclopentenyl)ethyl acetate, and the racemic and scalemic mixtures thereof.

The process for obtaining 2- (1,5,5-trimethyl-2-cyclopentenyl)ethyl acetate (3) by a multi-step conversion of the alkoxycarbonylmethyl group (i.e., $-CH_2C(=O)OR$) of the (1,5,5-trimethyl-2-cyclopentenyl)acetate compound (2) to a 2-acetoxyethyl group (i.e., $-CH_2CH_2OAc$) may be carried out by a combination of at least two known methods for converting a functional group.

As shown in the following reaction formula, an example of the multi-step conversion comprises a step of subjecting the (1,5,5-trimethyl-2-cyclopentenyl)acetate compound (2) to a reduction reaction with a reducing agent to obtain 2-(1,5,5-trimethyl-2-cyclopentenyl)ethyl alcohol (5), and, subsequently, a step of acetylating 2-(1,5,5-trimethyl-2-cyclopentenyl)ethyl alcohol (5) thus obtained to obtain 2-(1,5,5-trimethyl-2-cyclopentenyl)ethyl acetate (3) (hereinafter, referred to as Preparation Process 1) (see Example 2-1 and Example 2-5 below).

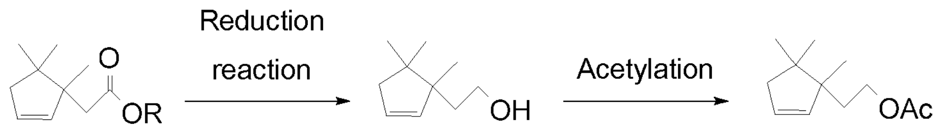


(2) (5) (3)

As shown in the following reaction formula, another example of the multi-step conversion comprising a step of hydrolyzing the (1,5,5-trimethyl-2-cyclopentenyl)acetate compound (2) to obtain (1,5,5-trimethyl-2-cyclopentenyl)acetic acid (6), a step of subjecting the (1,5,5-trimethyl-2-cyclopentenyl)acetic acid (6) to a reduction reaction with a reducing agent, as in Preparation Process 1, to obtain 2-(1,5,5-trimethyl-2-cyclopentenyl)ethyl alcohol (5), and, subsequently, a step of acetylating 2-(1,5,5-trimethyl-2-cyclopentenyl)ethyl alcohol (5) thus obtained to obtain 2-(1,5,5-trimethyl-2-cyclopentenyl)ethyl acetate (3) (hereinafter, referred to as Preparation Process 2) (see Example 2-2, Example 2-3, Example 2-4, and Example 2-5 below).

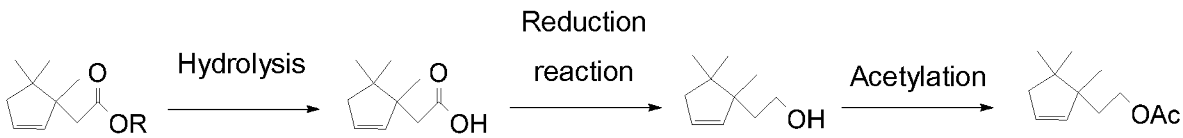


(2) (6) (5) (3)

(a) Preparation Process 1 will be described below.

The reduction reaction of the (1,5,5-trimethyl-2-cyclopentenyl)acetate compound (2) may be carried out using a reducing agent, and may be carried out with heating or cooling, if needed.

The reducing agent used in the reduction reaction may be a known reducing agent and is not specifically limited.

Examples of the reducing agent include hydrogen; boron compounds such as borane, alkylborane, dialkylborane, and bis(1,2-dimethylpropyl) borane; metal hydrides such as dialkylsilane, trialkylsilane, aluminum hydride, alkylaluminum hydride, dialkylaluminum hydride, sodium hydride, lithium hydride, potassium hydride, and calcium hydride; and metal hydride complexes such as sodium borohydride, lithium borohydride, potassium borohydride, sodium trimethoxyborohydride, lithium triethylborohydride, sodium aluminum hydride, lithium aluminum hydride, lithium trimethoxyaluminium hydride, lithium diethoxyaluminum hydride, lithium tri-t-butoxyaluminum hydride, and sodium bis(2-methoxyethoxy)aluminium hydride. The metal hydride complexes are preferred in view of the reactivity and/or the yield.

An amount of the reducing agent in the reduction reaction may be arbitrarily set depending on the reactivity of the reducing agent and/or the structure of the reducing agent and/or the reaction mechanism and is, for example, preferably from 0.2 mol to 10.0 mol, more preferably from 0.25 mol to 5.0 mol, per mol of the (1,5,5-trimethyl-2-cyclopentenyl)acetate compound (2) in view of the reactivity and/or the economy.

A solvent used in the reduction reaction may be any solvent that has no adverse effect on the reduction reaction. Examples of the solvent used in the reduction reaction include water; hydrocarbon solvents such as hexane, heptane, octane, benzene, toluene, and xylene; ether solvents such as diethyl ether, di-n-butyl ether, di-t-butyl ether, cyclopentyl methyl ether, diethyleneglycol diethyl ether, diethyleneglycol dimethyl ether, tetrahydrofuran, and 1,4-dioxane; halogen-based solvents such as methylene chloride and chloroform; alcoholic solvents such as methanol, ethanol, 1-propanol, and 2-propanol; nitrile solvents such as acetonitrile; and aprotic polar solvents such as N,N-dimethylformamide, N,N-dimethylacetamide, dimethyl sulfoxide, and hexamethylphosphoric triamide. The hydrocarbon solvents and the ether solvents are preferred in view of the reactivity.

The solvent may be used either alone or in combination thereof, if necessary, and may be optionally determined while considering the type and/or the reactivity of the (1,5,5-trimethyl-2-cyclopentenyl)acetate compound (2).

An amount of the solvent may be optionally determined while considering the reactivity and/or the solubility of the (1,5,5-trimethyl-2-cyclopentenyl)acetate compound (2) and is, for example, from 30 g to 10,000 g per mol of the (1,5,5-trimethyl-2-cyclopentenyl)acetate compound (2), more preferably from 50 g to 3,000 g, in view of the reactivity and/or the economy.

A reaction temperature of the reduction reaction may be carried out at a reaction temperature which does not have any adverse effect on the reduction reaction, and is preferably from -50°C to 200°C, more preferably from -25°C to 100°C, in view of the yield and/or the by-product formation.

The reaction time of the reduction reaction is preferably optimized, depending on the reactivity of the (1,5,5-trimethyl-2-cyclopentenyl)acetate compound (2), by monitoring the reaction progress with, for example, gas chromatography and/or thin layer chromatography to confirm the disappearance of the (1,5,5-trimethyl-2-cyclopentenyl)acetate compound (2) and is, for example, preferably from 1 hour to 168 hours, more preferably from 1 hour to 24 hours, even more preferably from 1 hour to 6 hours, in view of the yield and/or the formation of an impurity.

2-(1,5,5-Trimethyl-2-cyclopentenyl)ethyl alcohol (5) formed in the reduction reaction may be suitably isolated and/or purified in any purification method used in usual organic synthesis such as distillation at a reduced pressure and/or various chromatography. Distillation at a reduced pressure is preferred in view of the industrial economy. When 2-(1,5,5-trimethyl-2-cyclopentenyl)ethyl alcohol (5), which is the target substance, has a sufficient purity, the crude product comprising 2-(1,5,5-trimethyl-2-cyclopentenyl)ethyl alcohol (5) may be used as such without purification in a subsequent step.

2-(1,5,5-Trimethyl-2-cyclopentenyl)ethyl alcohol (5) to be obtained by the reduction reaction includes (R)-2-(1,5,5-trimethyl-2-cyclopentenyl)ethyl alcohol, (S)-2-(1,5,5-trimethyl-2-cyclopentenyl)ethyl alcohol, and the racemic and scalemic mixtures thereof.

The acetylation method on 2-(1,5,5-trimethyl-2-cyclopentenyl)ethyl alcohol (5) obtained by the reduction reaction may be a known acetylation method and is not specifically limited. Examples of the acetylation method include (i) an acetylation reaction between an alcohol compound and an acetylating agent, (ii) a dehydration reaction between an alcohol compound and an acetic acid, (iii) a transesterification reaction between an alcohol compound and an acetate compound, and (iv) converting an alcohol compound into an alkylating agent, followed by acetoxylation with an acetic acid or a metal acetate. 2-(1,5,5-Trimethyl-2-cyclopentenyl)ethyl acetate (3), which is the target compound of the present invention, may be prepared from 2-(1,5,5-trimethyl-2-cyclopentenyl)ethyl alcohol (5) as the alcohol compound.

(i) Acetylation reaction between an alcohol compound and an acetylating agent

The acetylation reaction between the alcohol compound and the acetylating agent is carried out generally in the presence of a base or a catalyst, and may be carried out with heating or cooling, if needed.

Examples of the acetylating agent include acetyl chloride, acetyl bromide or acetic anhydride.

An amount of the acetylating agent is preferably from 1.0 mol to 30.0 mol, more preferably from 1.0 mol to 5.0 mol, per mol of the alcohol compound in view of the economy.

When, for example, acetyl chloride, acetyl bromide or acetic anhydride is used as the acetylating agent, the acetylation may be carried out in the presence of a base.

Examples of the base in the acetylation reaction include amines such as triethylamine, pyridine, N,N-dimethylaminopyridine, and N,N-dimethylaniline; organometallic compounds such as n-butyl lithium, methyl lithium, and phenyl lithium; metal hydroxides such as sodium hydroxide and potassium hydroxide; and metal carbonates such as potassium carbonate, sodium carbonate, and sodium bicarbonate.

The base may be used either alone or in combination thereof, if necessary, and may be optionally determined while considering the type and/or the reactivity of the alcohol compound and/or the acetylating agent.

An amount of the base is preferably from 1.0 mol to 50.0 mol, more preferably from 1.0 to 10.0 mol, per mol of the alcohol compound in view of the economy.

When, for example, acetic anhydride is used as the acetylating agent, the acetylation may be carried out in the presence of a catalyst.

Examples of the catalyst include inorganic acids such as hydrochloric acid, hydrobromic acid, sulfuric acid, and nitric acid; sulfonic acids such as methanesulfonic acid, benzenesulfonic acid, and p-toluenesulfonic acid; Lewis acids such as aluminum trichloride, aluminum isopropoxide, zinc chloride, boron trifluoride, boron trichloride, tin tetrachloride, dibutyltin dichloride, titanium tetrachloride, and titanium (IV) isopropoxide; and metal acetates such as sodium acetate and potassium acetate.

The catalyst may be used either alone or in combination thereof, if necessary, and may be optionally determined while considering the type and/or the reactivity of the alcohol compound and/or the acetylating agent.

An amount of the catalyst is preferably from 0.001 mol to 1.0 mol, more preferably from 0.005 to 0.2 mol, per mol of the alcohol compound in view of the economy.

Optionally, a solvent may be used in the acetylation reaction.

The solvent may be any solvent that has no adverse effect on the alcohol compound, the acetylating agent, the base and/or the catalyst. Examples of the solvent include halogen-based solvents such as methylene chloride and chloroform; hydrocarbon solvents such as hexane, heptane, benzene, and toluene; ether solvents such as diethyl ether, tetrahydrofuran, 1,4-dioxane, and diethylene glycol dimethyl ether; nitrile solvents such as acetonitrile; ketone solvents such as acetone, methyl ethyl ketone, and diisobutyl ketone; ester solvents such as ethyl acetate and butyl acetate; and aprotic polar solvents such as N,N-dimethylformamide, N,N-dimethylacetamide, dimethyl sulfoxide, and hexamethylphosphoric triamide.

The solvent may be used either alone or in combination thereof, if necessary, and may be optionally determined while considering the type and/or the reactivity of the alcohol compound, the acetylating agent, the base and/or the catalyst.

An amount of the solvent is preferably more than 0 g to 10,000 g, more preferably more than 0 g to 5,000 g, per mol of the alcohol compound in view of the economy.

Note that the acetylation reaction may be carried out without a solvent, depending on the type of the alcohol compound, the acetylating agent, the base and/or the catalyst.

A reaction temperature of the acetylation reaction is preferably from -78°C to 120°C, more preferably from -30°C to 80°C, in view of the reactivity and/or the yield.

The reaction time of the acetylation reaction is preferably optimized, depending on the reactivity of the alcohol compound, the acetylating agent, the base and/or the catalyst, by monitoring the reaction progress with, for example, gas chromatography and/or thin layer chromatography to confirm the disappearance of the alcohol compound, and is, for example, preferably from 1 hour to 168 hours, more preferably from 1 hour to 24 hours, even more preferably from 1 hour to 18 hours, in view of the yield and/or the formation of an impurity.

(ii) Dehydration reaction between an alcohol compound and an acetic acid

The dehydration reaction between the alcohol compound and the acetic acid is carried out generally in the presence of a catalyst, and may be carried out with heating or cooling, if needed.

Examples of the catalyst in the dehydration reaction include acids or Lewis acids.

Examples of the acid as the catalyst include inorganic acids such as hydrochloric acid, hydrobromic acid, sulfuric acid, and nitric acid; and organic acids such as trichloroacetic acid, trifluoroacetic acid, methanesulfonic acid, benzenesulfonic acid, and p-toluenesulfonic acid. Examples of the Lewis acids as the catalyst include aluminum trichloride, dichloroaluminum ethoxide, aluminum ethoxide, aluminum isopropoxide, zinc diisopropoxide, zinc diethoxide, zinc dimethoxide, zinc chloride, boron trifluoride, boron trichloride, tin tetrachloride, dibutyltin dichloride, dibutyltin dimethoxide, dibutyltin oxide, titanium tetrachloride, titanium tetrabromide, titanium (IV) methoxide, titanium (IV) ethoxide, and titanium (IV) isopropoxide.

The catalyst may be used either alone or in combination thereof, if necessary, and may be optionally determined while considering the reactivity of the alcohol compound.

An amount of the catalyst in the dehydration reaction is preferably from 0.001 to 1.0 mol, more preferably from 0.05 to 0.1 mol, per mol of the alcohol compound in view of the economy and the reactivity.

The dehydration reaction may be carried out while removing water by-produced by the reaction. The dehydration reaction may be conducted, while azeotropically distillating off water and the reaction solvent at normal or reduced pressure , or with a dehydration agent such as anhydrous magnesium sulfate, molecular sieve, or dicyclohexylcarbodiimide added into the reaction system.

A solvent used in the dehydration reaction may be any solvent that has no adverse effect on the alcohol compound and/or the catalyst to be used. Examples of the solvent in the dehydration reaction include halogen-based solvents such as methylene chloride and chloroform; hydrocarbon solvents such as hexane, heptane, benzene, and toluene; ether solvents such as diethyl ether, tetrahydrofuran, 1,4-dioxane, and diethylene glycol dimethyl ether; nitrile solvents such as acetonitrile; ketone solvents such as acetone, methyl ethyl ketone, and diisobutyl ketone; and ester solvents such as ethyl acetate and butyl acetate.

The solvent may be used either alone or in combination thereof, if necessary, and may be optionally determined while considering the type and/or the reactivity of the alcohol compound and/or the catalyst.

An amount of the solvent in the dehydration reaction is preferably more than 0 g to 10,000 g, more preferably more than 0 g to 3,000 g, per mol of the alcohol compound in view of the economy.

Note that the dehydration reaction may be carried out without a solvent, depending on the type of the alcohol compound and/or the catalyst.

A reaction temperature of the dehydration reaction may be appropriately determined depending on the type of the alcohol compound and/or the catalyst to be used, and is preferably from -30°C to 200°C, more preferably 25°C to 100°C, in view of the reactivity and/or the yield. When the water by-produced by the dehydration reaction is azeotropically distilled off together with the solvent, the dehydration reaction is preferably carried out at a reaction temperature no less than the azeotropic point of the solvent and water at normal or reduced pressure.

The reaction time of the dehydration reaction is preferably optimized, depending on the type and/or the reactivity of the alcohol compound and/or the catalyst, by monitoring the reaction progress with, for example, gas chromatography and/or thin layer chromatography to confirm the disappearance of the alcohol compound, and is, for example, preferably from 1 hour to 168 hours, more preferably from 1 hour to 24 hours, even more preferably from 1 hour to 12 hours, in view of the yield and/or the formation of an impurity.

(iii) Transesterification reaction between an alcohol compound and an acetate compound

The transesterification reaction between the alcohol compound and the acetate compound is carried out generally in the presence of a catalyst. The alcohol formed from the acetate during the transesterification reaction may be removed at normal or reduced pressure to promote the reaction, and may be carried out with heating or cooling, if needed.

Examples of the acetate compound in the transesterification reaction include methyl acetate, ethyl acetate, propyl acetate, butyl acetate, and phenyl acetate. Among these acetates, methyl acetate and ethyl acetate are preferred in view of the economy, the reactivity and the ease of the removal of the alcohol by-produced from the acetate.

An amount of the acetate in the transesterification reaction is preferably from 1.0 mol to 30.0 mol, more preferably from 1.0 mol to 5.0 mol, per mol of the alcohol compound.

Examples of the catalyst in the transesterification reaction include acids such as hydrochloric acid, sulfuric acid, trifluoroacetic acid, methanesulfonic acid, benzenesulfonic acid, p-toluenesulfonic acid, and Amberlyst 15; alkali metal salts of alcohols such as sodium methoxide, sodium ethoxide, and potassium t-butoxide; carboxylic acid metal salts such as sodium acetate, potassium acetate, calcium acetate, tin acetate, zinc acetate and aluminum acetate; and Lewis acids such as aluminum trichloride, aluminum ethoxide, aluminum isopropoxide, zinc diisopropoxide, zinc diethoxide, zinc dimethoxide, zinc chloride, boron trifluoride, boron trichloride, tin tetrachloride, dibutyltin dichloride, dibutyltin dimethoxide, dibutyltin oxide, titanium tetrachloride, titanium tetrabromide, titanium (IV) methoxide, titanium (IV) ethoxide, and titanium (IV) isopropoxide.

An amount of the catalyst in the transesterification reaction is preferably from 0.001 to 1.0 mol, more preferably from 0.005 to 0.1 mol, per mol of the alcohol compound in view of the yield and the economy.

The solvent used in the transesterification reaction may be appropriately determined from solvents which do not have any adverse effect on the catalyst. Examples of the solvent used in the transesterification reaction include halogen-based solvents such as methylene chloride and chloroform; hydrocarbon solvents such as hexane, heptane, benzene, and toluene; ether solvents such as diethyl ether, tetrahydrofuran, 1,4-dioxane, and diethylene glycol dimethyl ether; nitrile solvents such as acetonitrile; ketone solvents such as acetone, methyl ethyl ketone, and diisobutyl ketone; and ester solvents such as ethyl acetate and butyl acetate.

The solvent may be used either alone or in combination thereof, if necessary, and may be optionally determined while considering the type and/or the reactivity of the alcohol compound, the acetate and/or the catalyst.

An amount of the solvent is preferably more than 0 g to 2,000 g, most preferably more than 0 g to 500 g, per mol of the alcohol compound in view of the economy.

Note that the transesterification reaction may be carried out without a solvent, depending on the type of the alcohol compound, the acetate and/or the catalyst.

A reaction temperature of the transesterification reaction may be appropriately determined depending on the type of the alcohol compound, the acetate and/or the catalyst. Generally, the reaction temperature is preferably from 0°C to 200°C, more preferably from 50°C to 150°C. When removing the alcohol produced as a by-product during the transesterification reaction to promote the reaction, the transesterification reaction is preferably carried out at a reaction temperature no less than the boiling point of the alcohol to be removed at normal or reduced pressure.

The reaction time of the transesterification reaction is preferably optimized, depending on the reactivity of the alcohol compound and/or the acetate, by monitoring the reaction progress with, for example, gas chromatography and/or thin layer chromatography to confirm the disappearance of the alcohol compound and/or the acetate. For example, the reaction time of the transesterification reaction is preferably from 1 hour to 168 hours, more preferably from 1 hour to

24 hours, even more preferably from 1 hour to 12 hours, in view of the yield and/or the formation of an impurity.

(iv) Conversion of an alcohol compound into an alkylating agent, followed by acetoxylation with an acetic acid or a metal acetate

The conversion of the alcohol compound into the alkylating agent, followed by acetoxylation with an acetic acid or a metal acetate are carried out with heating or cooling, if needed. Examples of the metal acetate include sodium acetate, potassium acetate.
Generally, the alcohol compound may be converted into an alkylating agent corresponding to the alcohol compound for example, halides such as chloride, bromide and iodide; sulfonate esters such as methanesulphonates, benzenesulphonates, and p-toluenesulphonates, and then, acetoxylation may be carried out. When carrying out the acetoxylation with an acetic acid, the acetoxylation is carried out in the presence of a base, and, when carrying out the acetoxylation with a metal acetate, the acetoxylation may be carried out without a base.
Examples of the base in the acetoxylation reaction include amines such as triethylamine, pyridine, N,N-dimethylaminopyridine, and dimethylaniline; organometallic compounds such as n-butyl lithium, methyl lithium, and phenyl lithium; metal hydroxides such as sodium hydroxide and potassium hydroxide; metal carbonates such as potassium carbonate, sodium carbonate, and sodium bicarbonate; and metal hydrides such as sodium hydride and potassium hydride.
The base may be used either alone or in combination thereof, if necessary, and may be optionally determined while considering the type and/or the reactivity of the alkylating agent.
An amount of the base is preferably from 1.0 mol to 50.0 mol per mol of the alkylating agent, and is more preferably from 1.0 to 10.0 mol per mol of the alkylating agent in view of the economy.
The solvent used in the acetoxylation reaction may be appropriately determined from any solvent which does not have any adverse effect on the acetoxylation reaction. Examples of the solvent used in the acetoxylation reaction include halogen-based solvents such as methylene chloride, and chloroform; hydrocarbon solvents such as hexane, heptane, benzene, and toluene; ether solvents such as diethyl ether, tetrahydrofuran, 1,4-dioxane, and ethylene glycol dimethyl ether; nitrile solvents such as acetonitrile; ketone solvents such as acetone, methyl ethyl ketone, and diisobutyl ketone; ester solvents such as ethyl acetate and butyl acetate; and aprotic polar solvents such as N,N-dimethylformamide, N,N-dimethylacetamide, dimethyl sulfoxide, and hexamethylphosphoric triamide.
The solvent may be used either alone or in combination thereof, if necessary, and may be optionally determined while considering the type and/or the reactivity of the alkylating agent and/or acetic acid or metal acetate.
An amount of solvent in the acetoxylation reaction is preferably more than 0 g to 5,000 g per mol of the alkylating agent, more preferably more than 0 g to 1,000 g in view of the economy.
A reaction temperature of the acetoxylation reaction is preferably from -30°C to 250°C, more preferably from 25°C to 180°C, in view of the reactivity and/or the yield.
The reaction time of the acetoxylation reaction is preferably optimized, depending on the reactivity of the alkylating agent and/or acetic acid or metal acetate, by monitoring the reaction progress with, for example, gas chromatography and/or thin layer chromatography to confirm the disappearance of the alkylating agent, and is, for example, preferably from 1 hour to 168 hours, more preferably from 1 hour to 24 hours, even more preferably from 1 hour to 12 hours, in view of the yield and/or the formation of an impurity.
2-(1,5,5-Trimethyl-2-cyclopentenyl)ethyl acetate (3) formed in the acetylation reaction of 2-(1,5,5-trimethyl-2-cyclopentenyl)ethyl alcohol (5) , as stated in the aforementioned (i) to (iv), may be suitably isolated and/or purified in any purification method used in usual organic synthesis such as distillation at a reduced pressure and/or various chromatography. Distillation at a reduced pressure is preferred in view of the industrial economy. When 2-(1,5,5-trimethyl-2-cyclopentenyl)ethyl acetate (3), which is the target substance, has a sufficient purity, the crude product comprising 2-(1,5,5-trimethyl-2-cyclopentenyl)ethyl acetate (3) may be used as such without purification.
(b) Next, the Preparation Process 2 will be described below.
The hydrolysis reaction from the (1,5,5-trimethyl-2-cyclopentenyl)acetate compound (2) to 2-(1,5,5-trimethyl-2-cyclopentenyl)ethyl acetate (3) may be carried out using a known method, and may be carried out with heating or cooling, if needed.
The hydrolysis reaction may be carried out, for example, in a basic condition in the presence of a base, in an acidic condition in the presence of an acid, or in a neutral condition in the presence of a salt or a halogenated silane.
Examples of the base used in the hydrolysis in a basic condition include hydroxide salts such as sodium hydroxide, lithium hydroxide, potassium hydroxide, and barium hydroxide; carbonates or bicarbonates such as sodium carbonate, potassium carbonate, sodium bicarbonate, and potassium bicarbonate; and metal alkoxides such as sodium methoxide, sodium ethoxide, sodium t-butoxide, lithium methoxide, lithium ethoxide, lithium t-butoxide, potassium methoxide, potassium ethoxide, and potassium t-butoxide.
The base may be used either alone or in combination thereof, if necessary, and may be optionally determined

while considering the type and/or the reactivity and/or the selectivity of the (1,5,5-trimethyl-2-cyclopentenyl)acetate compound (2).

Examples of the acid used in the hydrolysis in an acidic condition include inorganic acids such as hydrochloric acid, hydrobromic acid, sulfuric acid, and nitric acid; organic acids such as acetic acid, formic acid, oxalic acid, trifluoroacetic acid, methanesulfonic acid, benzenesulfonic acid, and p-toluenesulfonic acid; and Lewis acids such as aluminum trichloride, aluminum ethoxide, aluminum isopropoxide, boron trifluoride, boron trichloride, boron tribromide, tin tetrachloride, tin tetrabromide, dibutyltin dichloride, dibutyltin dimethoxide, dibutyltin oxide, titanium tetrachloride, titanium tetrabromide, titanium (IV) methoxide, titanium (IV) ethoxide, and titanium (IV) isopropoxide.

The acid, may be used either alone or in combination thereof, if necessary, and may be optionally determined while considering the type and/or the reactivity and/or the selectivity of the (1,5,5-trimethyl-2-cyclopentenyl)acetate compound (2).

Examples of the salt and the halogenated silane used in the hydrolysis in a neutral condition include salts such as lithium iodide, lithium bromide, sodium cyanide, potassium cyanide, lithium methanethiolate, and sodium benzenethiolate; and halogenated silanes such as trimethylsilyl iodide and trimethylsilyl bromide.

The salt or the halogenated silane may be used either alone or in combination thereof, if necessary, and may be optionally determined while considering the type and/or the reactivity and/or the selectivity of the (1,5,5-trimethyl-2-cyclopentenyl)acetate compound (2).

The hydrolysis reaction is preferably carried out in a basic condition, and is more preferably carried out by in the presence of a hydroxide salt, carbonate or bicarbonates in view of the yield and/or the formation of an impurity.

An amount of the base, acid, or salt or halogenated silane used in the hydrolysis reaction may be arbitrarily set in the range from a very low catalytic amount to a large excess, depending on the reactivity of the (1,5,5-trimethyl-2-cyclopentenyl)acetate compound (2) and is, for example, preferably from 0.1 mol to 50.0 mol, more preferably from 0.5 mol to 10.0 mol, per mol of the (1,5,5-trimethyl-2-cyclopentenyl)acetate compound (2) in view of the reaction time and/or by-production of an impurity.

A solvent used in the hydrolysis reaction may be any solvent that has no adverse effect on the hydrolysis reaction. Examples of the solvent used in the hydrolysis reaction include water; alcohol solvents such as methanol, ethanol, isopropyl alcohol, and t-butyl alcohol; ether solvents such as diethyl ether, di-n-butyl ether, di-t-butyl ether, tetrahydrofuran, 1,4-dioxane, and diethyleneglycol dimethyl ether; hydrocarbon solvents such as hexane, heptane, benzene, toluene, and xylene; aprotic polar solvents such as N,N-dimethylformamide, N,N-dimethylacetamide, 1,3-dimethyl-2-imidazolidinone, dimethyl sulfoxide, and hexamethylphosphoric triamide; and nitrile solvents such as acetonitrile. The alcohol solvents and the ether solvents are preferred in view of the reactivity.

The solvent may be used either alone or in combination thereof, if necessary, and may be optionally determined while considering the type and/or the reactivity of the (1,5,5-trimethyl-2-cyclopentenyl)acetate compound (2).

An amount of the solvent used may be optionally determined while considering the reactivity and/or the solubility of the (1,5,5-trimethyl-2-cyclopentenyl)acetate compound (2) and is, for example, preferably from 30 g to 20,000 g, more preferably from 500 g to 8,000 g, per mol of the (1,5,5-trimethyl-2-cyclopentenyl)acetate compound (2) in view of the reactivity and/or the economy.

A reaction temperature of the hydrolysis reaction may be optionally determined while considering the reactivity and/or the formation of an impurity of the (1,5,5-trimethyl-2-cyclopentenyl)acetate compound (2). The reaction temperature is preferably from -60°C to 250°C, more preferably from 0°C to 100°C, in view of the reactivity and/or the formation of an impurity.

The reaction time of the hydrolysis reaction is preferably optimized, depending on the reactivity of the (1,5,5-trimethyl-2-cyclopentenyl)acetate compound (2), by monitoring the reaction progress with, for example, gas chromatography and/or thin layer chromatography to confirm the disappearance of the (1,5,5-trimethyl-2-cyclopentenyl)acetate compound (2) which is a substrate. For example, the reaction time of the hydrolysis reaction is preferably from 1 hour to 168 hours, more preferably from 1 hour to 24 hours, even more preferably from 1 hour to 12 hours, in view of the yield and/or the formation of an impurity.

(1,5,5-Trimethyl-2-cyclopentenyl)acetic acid (6) obtained by the hydrolysis reaction may be treated by dissolving it in a basic condition to form a carboxylate salt in an aqueous layer, extracting the aqueous layer containing the carboxylate salt, layer-separating the organic layer, acidifying the resulting aqueous layer, and re-extracting the aqueous layer with an organic solvent.

(1,5,5-Trimethyl-2-cyclopentenyl)acetic acid (6) formed in the hydrolysis reaction may be suitably isolated and/or purified in any purification method used in usual organic synthesis such as distillation at a reduced pressure and/or various chromatography. Distillation at a reduced pressure is preferred in view of the industrial economy. When a target compound (1,5,5-trimethyl-2-cyclopentenyl)acetic acid (6) has a sufficient purity, the crude product containing (1,5,5-trimethyl-2-cyclopentenyl)acetic acid (6) may be used as such without purification in a subsequent step.

(1,5,5-Trimethyl-2-cyclopentenyl)acetic acid (6) includes (R)-(1,5,5-trimethyl-2-cyclopentenyl)acetic acid, (S)-(1,5,5-trimethyl-2-cyclopentenyl)acetic acid, and the racemic and scalemic mixtures thereof.

The method for subjecting (1,5,5-trimethyl-2-cyclopentenyl)acetic acid (6) to a reduction reaction with a reducing agent to obtain 2-(1,5,5-trimethyl-2-cyclopentenyl)ethyl alcohol (5) may be carried out in a similar manner as in the reduction of the (1,5,5-trimethyl-2-cyclopentenyl)acetate compound (2) into 2-(1,5,5-trimethyl-2-cyclopentenyl)ethyl alcohol (5) in the aforementioned Preparation Process 1.

The method for subjecting 2-(1,5,5-trimethyl-2-cyclopentenyl)ethyl alcohol (5) obtained by the reduction reaction to an acetylation reaction to obtain 2-(1,5,5-trimethyl-2-cyclopentenyl)ethyl acetate (3) is as described for the acetylation reaction in the aforementioned Preparation Process 1.

As stated above, the (1,5,5-trimethyl-2-cyclopentenyl)acetate compound (2) is obtained in an industrially readily applicable range of the reaction temperature without an ignitable starting material and an industrially expensive starting material by subjecting the haloacetaldehyde alkyl 3,4,4-trimethyl-2-cyclopentenyl acetal compound (1) to a dehydrohalogenation reaction in the presence of a base, followed by a rearrangement reaction and, then, the alkoxycarbonylmethyl group (i.e., $-CH_2C(=O)OR$) of the (1,5,5-trimethyl-2-cyclopentenyl)acetate compound (2) is subjected to a multi-step conversion to be converted into a 2-acetoxyethyl group (i.e., $-CH_2CH_2OAc$), whereby 2-(1,5,5-trimethyl-2-cyclopentenyl)ethyl acetate (3) is efficiently and industrially prepared.

## Examples

The present invention will be described with reference to the following Examples and Comparative Examples. It should be construed that the present invention is not limited to or by the Examples.

The term "purity" as used herein means an area percentage determined by gas chromatography (hereinafter referred to also as "GC"), unless otherwise specified.

The term "yield" is calculated from the area percentages determined by GC.

The yield was calculated by the following equation in consideration of purities (% GC) of a starting material and a product.

$$\text{Yield (\%)} = [(\text{mass of a product obtained in a reaction x \% GC})/\text{molecular mass of a product}] \div [(\text{mass of a starting material x \% GC})/\text{molecular mass of a starting material}]\} \text{ x } 100$$

GC conditions were as follows:
GC conditions for determination of "purity" and "product ratio": GC: Capillary gas chromatograph GC-2010 (Shimadzu Corporation); column: DB-5, 0.25 μm × 0.25 mmϕ × 30 m, carrier gas: He (1.55 mL/min); detector: FID; column temperature: 60°C, kept for 3 minutes, elevated by 10°C/min, up to 230°C.

As used herein, Et represents an ethyl group, $^{i}Pr$ represents an isopropyl group, and $^{n}Bu$ represents an n-butyl group.

## Synthesis Examples

The following Synthesis Example 1-1 to Synthesis Example 1-4 describe a process for preparing the haloacetaldehyde alkyl 3,4,4-trimethyl-2-cyclopentenyl acetal compound (1), as shown in the following reaction formula.

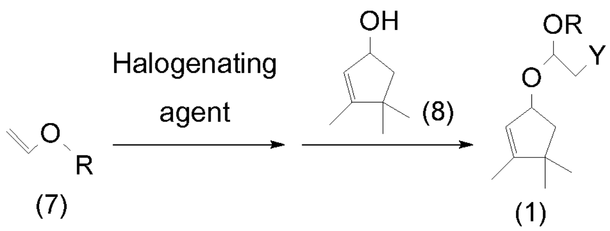

(8)
(7)
(1)

### Synthesis Example 1-1

Preparation of bromoacetaldehyde ethyl 3,4,4-trimethyl-2-cyclopentenyl acetal (1: R = Et; Y = Br)

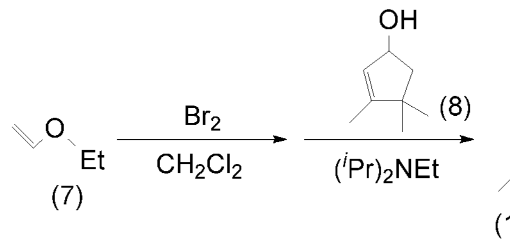


(7)

(1: R=Et; Y=Br)

Air in a reactor equipped with a stirrer, a condenser, and a thermometer was purged with nitrogen. Then, to the reactor were added bromine ($Br_2$) (26.36 g: 0.165 mol) and methylene chloride ($CH_2Cl_2$) (750.0 g), and the liquid temperature was lowered to -5°C to 0°C. Ethyl vinyl ether (7: R = Et) (12.98 g: 0.180 mol) was added dropwise to the mixture whose temperature was maintained at a liquid temperature of -5°C to 0°C over 90 minutes. After the completion of the dropwise addition, the reaction mixture was stirred at a liquid temperature of -5°C to 0°C for 30 minutes. After the completion of the stirring, diisopropylethylamine (($^i$Pr)$_2$NEt) (23.27 g: 0.180 mol) was added at a liquid temperature of -5°C to 0°C over 10 minutes. After the completion of the addition, 3,4,4-trimethyl-2-cyclopenten-1-ol (8) (18.93 g: 0.150 mol, purity 94.9%) was added dropwise at a liquid temperature of -10°C to -5°C over 1 hour. After the completion of the dropwise addition, the reaction mixture was stirred at a liquid temperature of -5°C to 0°C for 1 hour, and then at a liquid temperature of 20°C to 25°C for 3 hours.

After the stirring, an aqueous 3.5 wt% sodium bicarbonate solution (500.0 g) was added to the reaction mixture to quench the reaction. After the quenching, the reaction mixture was separated into an organic layer and an aqueous layer. The resulting organic layer was washed with water (300.0 g) and with an aqueous 10.0 wt% sodium chloride solution (300.0 g) in this order. The solvent was removed from the washed organic layer at a reduced pressure, and the crude product was then purified by distillation at a reduced pressure to obtain bromoacetaldehyde ethyl 3,4,4-trimethyl-2-cyclopentenyl acetal (1: R = Et; Y = Br) (36.84 g: 0.133 mol, yield 88.6%, purity 91.8%).

The following are various spectrum data of bromoacetaldehyde ethyl 3,4,4-trimethyl-2-cyclopentenyl acetal (1: R = Et; Y = Br) thus prepared.

Nuclear magnetic resonance spectrum: $^1$H-NMR (500 MHz, $CDCl_3$): δ 1.00 (1.5H, s), 1.00 (1.5H, s), 1.10 (1.5H, s), 1.11 (1.5H, s), 1.22 (1.5H, t, J = 6.9 Hz), 1.23 (1.5H, t, J = 7.1 Hz), 1.66 (1.5H, t, J = 1.6 Hz), 1.66 (1.5H, t, J = 1.5 Hz), 1.75 (0.5H, q, J = 4.4 Hz), 1.77 (0.5H, q, 4.4 Hz), 2.02 (0.5H, q, J = 5.7 Hz), 2.05 (0.5H, q, J = 5.7 Hz), 3.32-3.37 (m, 2H), 3.53-3.62 (1H, m), 3.63-3.73 (1H, m), 4.62-4.68 (1H, m), 4.71 (0.5H, t, J = 2.9 Hz), 4.73 (0.5H, t, J = 3.1 Hz), 5.35 (1H, br) ppm. $^{13}$C-NMR (126 MHz, $CDCl_3$): δ 12.29, 15.13, 15.22, 27.31, 27.35, 27.73, 27.82, 32.29, 32.42, 45.09, 45.16, 47.12, 47.99, 61.34, 61.89, 79.95, 80.02, 100.53, 101.00, 123.24, 123.58, 153.88, 153.93 ppm.

Mass spectrum EI (70 eV): m/z 152, 151, 125, 123, 93, 91, 83, 81, 79, 77, 72, 57, 43, 29.

Infrared absorption spectrum (D-ATR): ν (cm$^{-1}$) 583, 683, 829, 892, 1032, 1055, 1115, 1190, 1223, 1338, 1361, 1376, 1437, 1465, 1653, 2866, 2929, 2956, 3046.

Synthesis Example 1-2

Preparation of bromoacetaldehyde ethyl 3,4,4-trimethyl-2-cyclopentenyl acetal (1: R = Et; Y = Br)

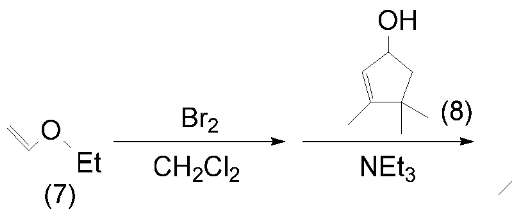


(7)

(1: R=Et; Y=Br)

The procedures of Synthesis Example 1-1 were repeated with the exception that triethylamine ($NEt_3$) (18.21 g: 0.180 mol) was used instead of diisopropylethylamine (($^i$Pr)$_2$NEt) as a base, so that obtained was bromoacetaldehyde ethyl 3,4,4-trimethyl-2-cyclopentenyl acetal (1: R = Et; Y = Br) (30.60 g: 0.110 mol, yield 73.3%, purity 89.2%) was obtained.

Various spectrum data of bromoacetaldehyde ethyl 3,4,4-trimethyl-2-cyclopentenyl acetal (1: R = Et; Y = Br) thus prepared were the same as those determined in Synthesis Example 1-1.

Synthesis Example 1-3

Preparation of bromoacetaldehyde isopropyl 3,4,4-trimethyl-2-cyclopentenyl acetal (1: R = $^{i}$Pr; Y = Br)

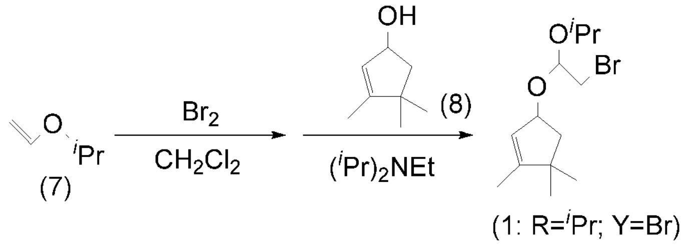


The procedures of Synthesis Example 1-1 were repeated with the exception that isopropyl vinyl ether (7: R = $^{i}$Pr) (15.50 g: 0.180 mol) was used instead of ethyl vinyl ether (7: R = Et), so that obtained was bromoacetaldehyde isopropyl 3,4,4-trimethyl-2-cyclopentenyl acetal (1: R= $^{i}$Pr; Y = Br) (41.19 g: 0.141 mol, yield 94.3%, purity 87.8%).

The following are various spectrum data of bromoacetaldehyde isopropyl 3,4,4-trimethyl-2-cyclopentenyl acetal (1: R = $^{i}$Pr; Y = Br) thus prepared.

Nuclear magnetic resonance spectrum: $^{1}$H-NMR (500 MHz, $CDCl_3$): δ 0.97 (1.5H, s), 0.97 (1.5H, s), 1.05 (1.5H, s), 1.05 (1.5H, s), 1.10 (3H, d, J = 6.0 Hz), 1.12 (3H, dd, J = 6.3, 3.0 Hz), 1.62-1.65 (4H, m), 1.97 (0.5H, dd, J = 13.2, 7.0 Hz), 2.02 (0.5H, dd, J = 13.2, 7.0 Hz), 3.35-3.41 (2H, m), 3.82-3.86 (1H, m), 4.62 (1H, m), 4.69 (0.5H, t, J = 5.1 Hz), 4.71 (0.5H, t, J = 5.1), 5.35 (1H, d, 22.8 Hz) ppm. $^{13}$C-NMR (126 MHz, $CDCl_3$): δ 11.97, 12.02, 22.14, 22.35, 23.02, 23.06, 26.97, 27.57, 27.58, 34.00, 34.04, 44.51, 44.59, 46.96, 47.69, 68.39, 68.51, 78.23, 78.24, 98.62, 98.78, 123.96, 124.25, 152.10, 152.13 ppm.

Mass spectrum EI (70 eV): m/z 168, 167, 151, 125, 110, 109, 93, 91, 77, 58, 43, 27.

Infrared absorption spectrum (D-ATR): ν ($cm^{-1}$) 681, 829, 1026, 1124, 1171, 1202, 1337, 1380, 1421, 1437, 1466, 2867, 2929, 2969, 3045.

Synthesis Example 1-4

Preparation of bromoacetaldehyde n-butyl 3,4,4-trimethyl-2-cyclopentenyl acetal (1: R = $^{n}$Bu; Y = Br)

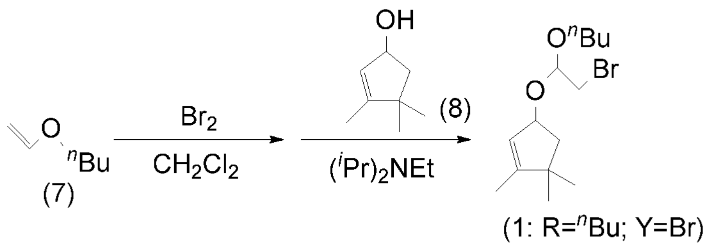


The procedures of Synthesis Example 1-1 were repeated with the exception that n-butyl vinyl ether (7: R = $^{n}$Bu) (18.03 g: 0.180 mol) was used instead of ethyl vinyl ether (7: R = Et), so that obtained was bromoacetaldehyde n-butyl 3,4,4-trimethyl-2-cyclopentenyl acetal (1: R = $^{n}$Bu; Y = Br) (34.62 g: 0.113 mol, yield 75.6%, purity 84.0%).

The following are various spectrum data of bromoacetaldehyde n-butyl 3,4,4-trimethyl-2-cyclopentenyl acetal (1: R = $^{n}$Bu; Y = Br) thus prepared.

Nuclear magnetic resonance spectrum: $^{1}$H-NMR (500 MHz, $CDCl_3$): δ 0.88 (3H, t, J = 7.5 Hz), 0.97 (3H, s), 1.05 (3H, s), 1.34 (2H, sextd, J = 7.5, 2.4 Hz), 1.46-1.51 (2H, m), 1.63-1.66 (4H, m), 1.98 (0.5H, q, J = 6.6 Hz), 2.01 (0.5H, q, J = 6.6 Hz), 3.39-3.47 (3H, m), 3.50-3.57 (1H, m), 4.60-4.62 (1H, m), 4.67 (1H, q, J = 5.6 Hz), 5.33 (0.5H, t, J = 1.5 Hz), 5.37 (0.5H, t, J = 1.5 Hz)ppm. $^{13}$C-NMR (126 MHz, $CDCl_3$): δ 11.97, 12.02, 13.69, 18.78, 26.95, 27.56, 31.29, 31.31, 33.25, 33.29, 44.51, 44.59, 46.87, 47.55, 65.03, 65.45, 79.11, 79.21, 99.94, 100.23, 123.82, 124.22, 152.20, 152.31 ppm.

Mass spectrum EI (70 eV): m/z 181, 179, 151, 125, 109, 93, 91, 77, 57, 41.

Infrared absorption spectrum (D-ATR): ν ($cm^{-1}$) 684, 829, 1036, 1114, 1187, 1224, 1338, 1360, 1377, 1435,

1465, 1653, 2869, 2933, 2957, 3047.

Example 1

The following Example 1-1 to Example 1-6 describe a process for preparing the (1,5,5-trimethyl-2-cyclopentenyl)acetate compound (2) from the haloacetaldehyde alkyl 3,4,4-trimethyl-2-cyclopentenyl acetal compound (1), as shown in the following reaction formula.

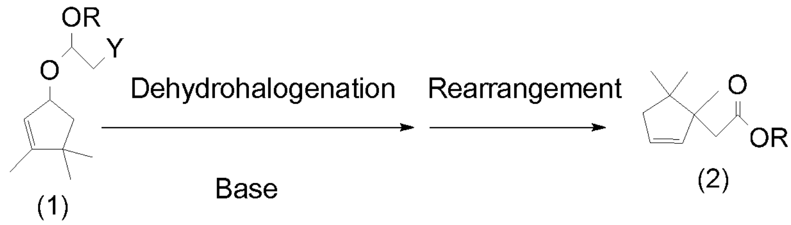


(1) (2)

Example 1-1

Preparation of ethyl (1,5,5-trimethyl-2-cyclopentenyl)acetate (2: R = Et)

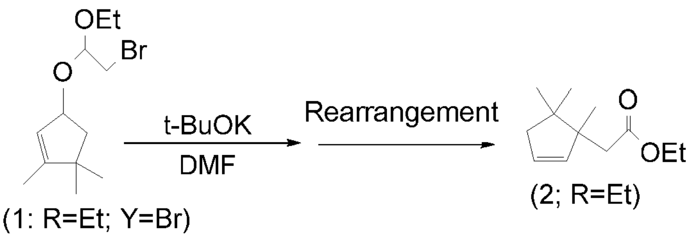


(1: R=Et; Y=Br) (2; R=Et)

Air in a reactor equipped with a stirrer, a condenser, and a thermometer was purged with nitrogen. Then, to the reactor were added bromoacetaldehyde ethyl 3,4,4-trimethyl-2-cyclopentenyl acetal (1: R = Et; Y = Br) (27.72 g: 0.100 mol, purity 92.0%) obtained according to Synthesis Example 1-1 and N,N-dimethylformamide (DMF) (280.0 g) and then cooled to a liquid temperature of 0°C to 5°C. Potassium t-butoxide (t-BuOK) (12.34 g: 0.110 mol) was added to the mixture whose temperature was maintained at a liquid temperature of 0°C to 5°C over 30 minutes. After the completion of the addition, the reaction mixture was allowed at a liquid temperature of 20 to 25°C for 4 hours.

After the reaction, the reaction mixture was heated to 100°C and stirred for 5 hours. After the completion of the stirring, the reaction mixture was cooled to 0°C to 5°C, and water (250.0 g) was added to the reactor to quench the reaction. Diethyl ether (300.0 g) was further added to the reactor to extract and layer-separate the mixture into an organic layer and an aqueous layer. The organic layer was washed with an aqueous 10 wt% sodium chloride solution (300.0 g). The solvent was removed from the washed organic layer at a reduced pressure, and the crude product was then purified by silica gel column chromatography to obtain ethyl (1,5,5-trimethyl-2-cyclopentenyl)acetate (2: R = Et) (9.76 g: 0.050 mol, yield 49.7%, purity 82.2%).

The following are various spectrum data of ethyl (1,5,5-trimethyl-2-cyclopentenyl)acetate (2: R = Et) thus prepared.

Nuclear magnetic resonance spectrum: $^1$H-NMR (500 MHz, $CDCl_3$): δ 0.92 (3H, s), 0.96 (3H, s), 0.96 (3H, s), 1.25 (3H, t, J = 7.3 Hz), 2.09 (1H, dt, J = 15.7, 2.1 Hz), 2.16 (1H, dt, J = 16.1, 2.2 Hz), 2.18 (1H, d, J = 13.4 Hz), 2.31 (1H, d, J = 13.4 Hz), 4.11 (2H, q, J = 7.1 Hz), 5.62 (1H, dt, J = 5.7, 2.4 Hz), 5.77 (1H, dt, J = 5.7, 1.9 Hz) ppm. $^{13}$C-NMR (126 MHz, $CDCl_3$): δ 14.27, 19.79, 23.95, 24.47, 40.78, 44.04, 46.64, 49.81, 59.97, 127.77, 138.79, 173.07 ppm.

Mass spectrum EI (70 eV): m/z 196 ($M^+$), 181, 167, 150, 135, 122, 109, 108, 107, 93, 91, 81, 79, 77, 67, 55, 41, 28.

Infrared absorption spectrum (D-ATR): ν ($cm^{-1}$) 716, 742, 956, 1034, 1131, 1187, 1213, 1294, 1336, 1367, 1448, 1463, 1734, 2843, 2872, 2968, 2052.

Example 1-2

Preparation of ethyl (1,5,5-trimethyl-2-cyclopentenyl)acetate (2: R = Et)

With the exception that the reaction mixture was heated to 140°C and stirred for 9 hours in place of the reaction mixture of Example 1-1 being heated to 100°C and stirred for 5 hours, Example 1-2 was conducted in the same manner as Example 1-1. As a result, ethyl (1,5,5-trimethyl-2-cyclopentenyl)acetate (2: R=Et) (9.38 g: 0.048 mol, yield 47.8%, purity 80.0%) was obtained.
The various spectrum data of the ethyl (1,5,5-trimethyl-2-cyclopentenyl)acetate (2: R = Et) thus prepared were the same as those determined in Example 1-1.

Example 1-3

Preparation of ethyl (1,5,5-trimethyl-2-cyclopentenyl)acetate (2: R = Et)

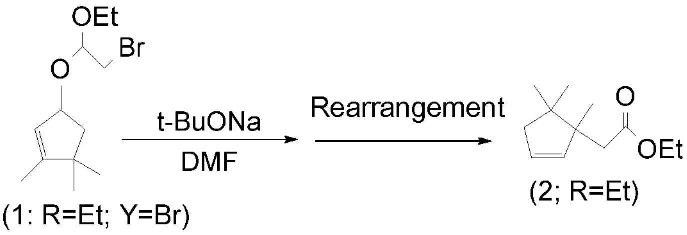


(1: R=Et; Y=Br)

(2; R=Et)

The procedures of Example 1-1 were repeated with the exception that sodium t-butoxide (t-BuONa) (10.57 g: 0.110 mol) was used instead of potassium t-butoxide (t-BuOK) as a base used in Example 1-1, so that obtained was ethyl (1,5,5-trimethyl-2-cyclopentenyl)acetate (2: R = Et) (8.62 g: 0.044 mol, yield 43.9%, purity 83.7%).
The various spectrum data of the ethyl (1,5,5-trimethyl-2-cyclopentenyl)acetate (2: R = Et) thus prepared were the same as these determined in Example 1-1.

Example 1-4

Preparation of ethyl (1,5,5-trimethyl-2-cyclopentenyl)acetate (2: R = Et)

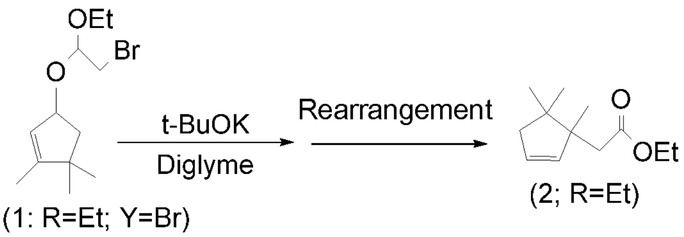


(1: R=Et; Y=Br)

(2; R=Et)

The procedures of Example 1-1 were repeated with the exception that diethyleneglycol dimethyl ether (Diglyme) (280.0 g) was used instead of N,N-dimethylformamide (DMF) as a solvent used in Example 1-1, so that obtained was ethyl (1,5,5-trimethyl-2-cyclopentenyl)acetate (2: R = Et) (9.50 g:0.048 mol, yield 48.4%, purity 84.0%).
The various spectrum data of the ethyl (1,5,5-trimethyl-2-cyclopentenyl)acetate (2: R = Et) thus prepared were the same as those determined in Example 1-1.

Example 1-5

Preparation of isopropyl (1,5,5-trimethyl-2-cyclopentenyl)acetate (2: R =$^i$Pr)

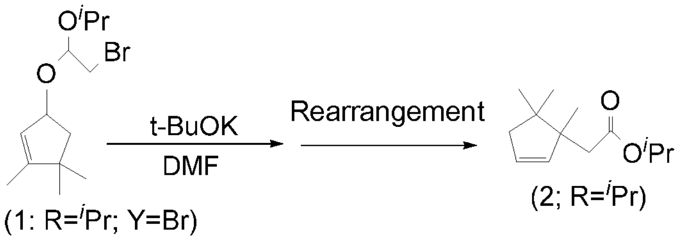


(1: R=$^i$Pr; Y=Br)

(2; R=$^i$Pr)

The procedures of Example 1-1 were repeated with the exception that bromoacetaldehyde isopropyl 3,4,4-trimethyl-2-cyclopentenyl acetal (1: R = $^i$Pr; Y = Br) (29.12 g: 0.100 mol, purity 95.0%) obtained according to Synthesis Example 1-3 was used instead of bromoacetaldehyde ethyl 3,4,4-trimethyl-2-cyclopentenyl acetal (1: R = Et; Y = Br) as a reaction substrate used in Example 1-1, and that distillation at a reduced pressure was used instead of purification by silica gel column chromatography in Example 1-1, so that obtained was isopropyl (1,5,5-trimethyl-2-cyclopentenyl)acetate (2: R = $^i$Pr) (6.37 g: 0.030 mol, yield 30.3%, purity 84.5%).

The following are various spectrum data of isopropyl (1,5,5-trimethyl-2-cyclopentenyl)acetate (2: R = $^i$Pr) thus prepared.

Nuclear magnetic resonance spectrum: $^1$H-NMR (500 MHz, $CDCl_3$): δ 0.93 (3H, s), 0.97 (6H, s), 1.24 (6H, d, 6.0 Hz), 2.09 (1H, dt, J = 16.0, 1.8 Hz), 2.15-2.19 (2H, m), 2.29 (1H, d, 13.2 Hz), 5.01 (1H, sep, J = 6.6 Hz), 5.63 (1H, dt, J = 5.7, 2.4 Hz), 5.79 (1H, dt, J = 6.0, 1.8 Hz) ppm. $^{13}$C-NMR (126 MHz, $CDCl_3$): δ 19.98, 22.03, 22.05, 24.05, 24.72, 41.22, 44.22, 46.79, 50.03, 67.43, 127.86, 139.00, 172.80 ppm.

Mass spectrum EI (70 eV): m/z 210 ($M^+$), 167, 153, 135, 121, 109, 108, 107, 93, 91, 81, 67, 55, 43, 27.

Infrared absorption spectrum (D-ATR): ν ($cm^{-1}$) 716, 741, 964, 1108, 1182, 1215, 1278, 1293, 1319, 1374, 1386, 1450, 1468, 1687, 1730, 2874, 2935, 2974, 3053.

Example 1-6

Preparation of n-butyl (1,5,5-trimethyl-2-cyclopentenyl)acetate (2: R = $^n$Bu)

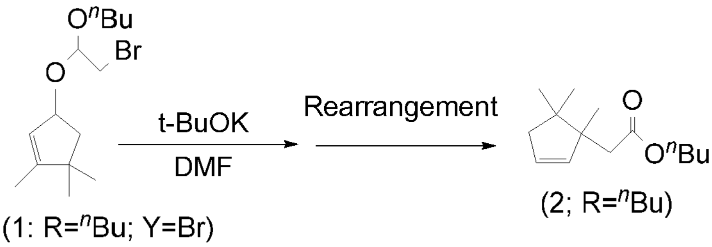


(1: R=$^n$Bu; Y=Br)

(2; R=$^n$Bu)

The procedures of Example 1-1 were repeated with the exception that bromoacetaldehyde n-butyl 3,4,4-trimethyl-2-cyclopentenyl acetal (1: R = $^n$Bu; Y = Br) (30.53 g: 0.100 mol, purity 84.0%) obtained in Synthesis Example 1-4 was used instead of bromoacetaldehyde ethyl 3,4,4-trimethyl-2-cyclopentenyl acetal (1: R = Et; Y = Br) as a reaction substrate used in Example 1-1, and that the resulting crude product was purified by distillation at a reduced pressure instead of silica gel chromatography used in Example 1-1, so that obtained was n-butyl (1,5,5-trimethyl-2-cyclopentenyl)acetate (2: R = $^n$Bu) (4.98 g: 0.022 mol, yield 22.2%, purity 64.5%).

The following are various spectrum data of n-butyl (1,5,5-trimethyl-2-cyclopentenyl)acetate (2: R = $^n$Bu) thus prepared.

Nuclear magnetic resonance spectrum: $^1$H-NMR (500 MHz, $CDCl_3$): δ 0.93-0.95 (6H, m), 0.97 (6H, s), 1.35-1.42 (3H, m), 1.61 (3H, quin, J = 7.4 Hz), 2.05 (1H, d, J = 15.6 Hz), 2.16-2.21 (2H, m), 2.32 (1H, d, J = 13.2 Hz), 4.06 (2H, t, J = 6.6 Hz), 5.64 (1H, dt, J = 5.7, 2.6 Hz), 5.78 (1H, d, J = 6.0 Hz) ppm. $^{13}$C-NMR (126 MHz, $CDCl_3$): δ 13.85, 19.37, 20.00, 24.11, 24.65, 30.85, 41.00, 44.21, 46.81, 49.98, 64.17, 127.96, 138.96, 173.41 ppm.

Mass spectrum EI (70 eV): m/z 224 ($M^+$), 209, 167, 153, 135, 122, 109, 108, 107, 93, 81, 67, 55, 41, 29.

Infrared absorption spectrum (D-ATR): ν ($cm^{-1}$) 716, 965, 1023, 1072, 1131, 1186, 1212, 1277, 1293, 1341, 1365, 1373, 1468, 1734, 2873, 2934, 2960, 3053.

Example 2

The following Example 2-1 to Example 2-5 describe a process for preparing 2-(1,5,5-trimethyl-2-cyclopentenyl)ethyl acetate (3) from the (1,5,5-trimethyl-2-cyclopentenyl)acetate compound (2), as shown in the following reaction formula.

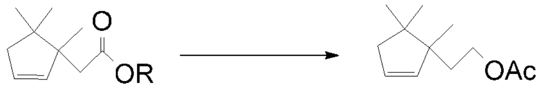

(2) (3)

Example 2-1

Preparation of 2-(1,5,5-trimethyl-2-cyclopentenyl)ethyl alcohol (5) (according to the aforementioned Preparation Process 1)

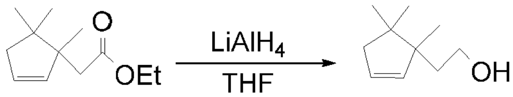


(2: R=Et) (5)

Air in a reactor equipped with a stirrer, a condenser, and a thermometer was purged with nitrogen. Then, to the reactor were added lithium aluminum hydride ($LiALH_4$) (8.35 g: 0.220 mol) and tetrahydrofuran (THF) (230.0 g), and mixture was stirred at 20 to 25°C for 2 hours to disperse lithium aluminum hydride. The temperature of the dispersion was adjusted to 0°C to 5°C, and to the reactor were added dropwise a solution of ethyl (1,5,5-trimethyl-2-cyclopentenyl)acetate (2: R = Et) (39.26 g: 0.200 mol) obtained in Example 1-1 in tetrahydrofuran (60.0 g) at a liquid temperature of 10°C to 15°C over 0.5 hour. After the completion of the dropwise addition, the reaction mixture was stirred at a liquid temperature of 10°C to 15°C for 1 hour, and then at a liquid temperature of 25°C to 30°C for 2 hours. The reaction mixture was cooled to no more than 5°C and, then, water (9.5 g), an aqueous 10 wt% sodium hydroxide solution (38.0 g), and tetrahydrofuran (30.0 g) were added dropwise in this order to the reactor at a liquid temperature of 0°C to 15°C. After the completion of the dropwise addition, the reaction mixture was stirred at a liquid temperature of 25°C to 35°C for 1 hour. After the stirring, the reaction mixture was filtered, and the solvent was removed from the filtrate at a reduced pressure to obtain 2-(1,5,5-trimethyl-2-cyclopentenyl)ethyl alcohol (5) (28.14 g: 0.182 mol, yield 91.2%, purity 86.7%). 2-(1,5,5-Trimethyl-2-cyclopentenyl)ethyl alcohol (5) thus prepared had a purity so as to allow the product (5) to be used as such without purification in the subsequent process.

The following are various spectrum data of 2-(1,5,5-trimethyl-2-cyclopentenyl)ethyl alcohol (5) thus prepared.

Nuclear magnetic resonance spectrum: $^1$H-NMR (500 MHz, $CDCl_3$): $\delta$ 0.87 (3H, s), 0.92 (3H, s), 0.94 (3H, s), 1.49-1.68 (3H, m), 2.11 (1H, d, J = 2.3 Hz), 2.12 (1H, d, J = 1.9 Hz), 3.65-3.77 (2H, m), 5.55 (1H, dt, J = 5.8, 1.9 Hz), 5.61 (1H, dt, J = 5.8, 2.3 Hz) ppm. $^{13}$C-NMR (126 MHz, $CDCl_3$): $\delta$ 19.43, 23.91, 24.72, 39.20, 44.00, 46.78, 49.45, 60.68, 128.17, 139.09 ppm.

Mass spectrum EI (70 eV): m/z 154 ($M^+$), 139, 121, 109, 95, 93, 81, 79, 67, 55, 41, 31.

Infrared absorption spectrum (D-ATR): ν ($cm^{-1}$) 716, 740, 759, 1021, 1050, 1080, 1129, 1364, 1451, 2843, 2960, 3050, 3326.

Example 2-2

Preparation of (1,5,5-trimethyl-2-cyclopentenyl)acetic acid (6) (according to the aforementioned Preparation Process 2)

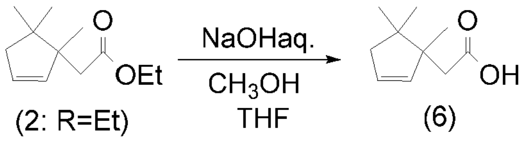


(2: R=Et) (6)

Air in a reactor equipped with a stirrer, a condenser, and a thermometer was purged with nitrogen. Then, to the reactor were added ethyl (1,5,5-trimethyl-2-cyclopentenyl)acetate (2: R = Et) (19.63 g: 0.100 mol, purity 82.2%) obtained according to Example 1-1, methanol (70.0 g), and tetrahydrofuran (THF) (150.0 g), and then heated to a liquid temperature of 50°C. An aqueous 5 wt% sodium hydroxide solution (NaOH aq.) (380.0 g: 0.475 mol) was added dropwise to the reactor at a liquid temperature of 50°C to 55°C over 2 hours. After the completion of the dropwise addition, the mixture was stirred at a liquid temperature of 55 to 60°C for 10 hours.

After the completion of the stirring, the reaction mixture was cooled to a liquid temperature of 25°C to 30°C, extracted with n-hexane (100.0 g), and layer-separated into an organic layer and an aqueous layer. To the aqueous layer was added dropwise 20 wt% hydrochloric acid (157.0 g) at a liquid temperature of 0°C to 10°C to make the aqueous layer acidic. The mixture was then extracted with n-hexane (150.0 g) and layer-separated into an organic layer and an aqueous layer. The obtained organic layer was washed twice with an aqueous 15 wt% sodium chloride solution (500.0 g). The solvent was removed from the washed organic layer at a reduced pressure to obtain (1,5,5-trimethyl-2-cyclopentenyl)acetic acid (6) (16.76 g: 0.099 mol, yield 99.6%, purity 93.2%).

(1,5,5-Trimethyl-2-cyclopentenyl)acetic acid (6) thus prepared had a purity so as to allow the product (6) to be used as such without purification in a subsequent process.

The following are various spectrum data of (1,5,5-trimethyl-2-cyclopentenyl)acetic acid (6) thus prepared.

Nuclear magnetic resonance spectrum: $^1$H-NMR (500 MHz, $CDCl_3$): $\delta$ 0.94 (3H, s), 0.97 (3H, s), 1.02 (3H, s), 2.12 (1H, dt, J = 16.1, 2.0 Hz), 2.18 (1H, dt, J = 16.1, 2.3 Hz), 2.23 (1H, d, J = 13.8 Hz), 2.36 (1H, d, J = 13.8 Hz), 5.66 (1H, dt, J = 6.1, 2.4 Hz), 5.80 (1H, dt, J = 6.1, 1.9 Hz), 11.53 (1H, br) ppm. $^{13}$C-NMR (126 MHz, $CDCl_3$): $\delta$ 19.65, 24.02, 24.37, 40.37, 44.13, 46.58, 49.70, 128.14, 138.44, 179.96 ppm.

Mass spectrum EI (70 eV): m/z 168 ($M^+$), 153, 135, 109, 93, 91, 79, 67, 55, 41, 27.

Infrared absorption spectrum (D-ATR): ν ($cm^{-1}$) 661, 716, 741, 954, 1137, 1199, 1235, 1295, 1374, 1386, 1409, 1449, 1706, 2844, 2966, 3053.

Example 2-3

Preparation of (1,5,5-trimethyl-2-cyclopentenyl)acetic acid (6) (according to the aforementioned Preparation Process 2)

The crude product (9.81 g: 0.050 mol, purity 41.8%) of ethyl (1,5,5-trimethyl-2-cyclopentenyl)acetate (2: R = Et) was obtained by the same procedures as in Example 1-1. Next, the same process procedures as in Example 2-2 were carried out on the obtained crude product. As a result, (1,5,5-trimethyl-2-cyclopentenyl)acetic acid (6) (8.09 g: 0.048 mol, yield 96.2%, purity 96.1%) was obtained. The (1,5,5-trimethyl-2-cyclopentenyl)acetic acid (6) thus prepared had a purity which allows to use the compound (6) to be used as such without purification in a subsequent process .

The various spectrum data of the (1,5,5-trimethyl-2-cyclopentenyl)acetic acid (6) thus prepared were the same as those determined in Example 2-2.

Further, according to Example 2-3, even when the purity of ethyl (1,5,5-trimethyl-2-cyclopentenyl)acetate (2: R = Et) is low, an increase in a purity of a resulting (1,5,5-trimethyl-2-cyclopentenyl)acetic acid (6) is possible on account of an aqueous sodium hydroxide solution used in the hydrolysis which works to conduct alkali extraction.

Example 2-4

Preparation of 2-(1,5,5-trimethyl-2-cyclopentenyl)ethyl alcohol (5) (according to the aforementioned Preparation Process 2)

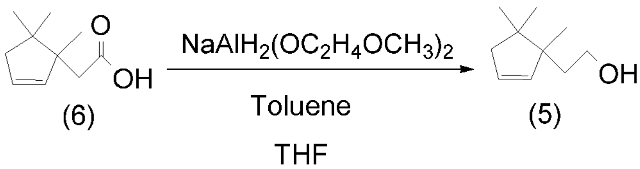


(6) (5)

Air in a reactor equipped with a stirrer, a condenser, and a thermometer was purged with nitrogen. Then, to the reactor were added (1,5,5-trimethyl-2-cyclopentenyl)acetic acid (6) (33.64 g: 0.200 mol, purity: 96.1%) obtained according to Example 2-3, and tetrahydrofuran (THF) (300.0 g), and then cooled to a liquid temperature of -5°C to 0°C. A toluene solution (115.53 g: 0.400 mol) of 70 wt% sodium bis(2-methoxyethoxy)aluminium hydride ($NaAlH_2(OC_2H_4OCH_3)_2$) was added dropwise to the reactor at liquid temperature of 0°C to 5°C over 2 hours. After the completion of the dropwise addition, the reaction mixture was stirred at a liquid temperature of 20°C to 25°C for 1 hour, and further at a liquid temperature of 55°C to 60°C for 6 hours. The reaction mixture was cooled to no more than 5°C and, then, an aqueous 10 wt% sodium hydroxide solution (320.0 g) was added dropwise to the reactor at a liquid temperature of 5°C to 15°C. After the completion of the dropwise addition, the reaction mixture was stirred at a liquid temperature of 35°C to 40°C for 1 hour.

After the completion of the stirring, the reaction mixture was layer-separated into an organic layer and an aqueous layer. The obtained organic layer was washed twice with an aqueous 10 wt% sodium chloride solution (200.0 g). The solvent was removed from the washed organic layer at a reduced pressure to obtain 2-(1,5,5-trimethyl-2-cyclopentenyl)ethyl alcohol (5) (28.63 g: 0.186 mol, yield 92.8%, purity 87.8%). 2-(1,5,5-Trimethyl-2-cyclopentenyl)ethyl alcohol (5) thus prepared had a purity which allows the product (5) to be used as such without purification in a subsequent process.

The various spectrum data of 2-(1,5,5-trimethyl-2-cyclopentenyl)ethyl alcohol (5) thus prepared was the same as those determined in Example 2-1.

Example 2-5

Preparation of 2-(1,5,5-trimethyl-2-cyclopentenyl)ethyl acetate (3) (common for both the aforementioned Preparation Process 1 and the aforementioned Preparation Process 2)

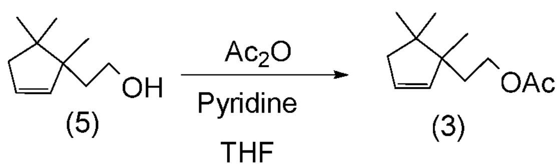


Air in a reactor equipped with a stirrer, a condenser, and a thermometer was purged with nitrogen. Then, to the reactor were added 2-(1,5,5-trimethyl-2-cyclopentenyl)ethyl alcohol (5) (30.85 g: 0.200 mol, purity: 86.7%) obtained according to Example 2-4, pyridine (31.64 g: 0.400 mol), and tetrahydrofuran (THF) (230.0 g), and then cooled to a liquid temperature of 5°C to 10°C. Acetic anhydride ($(CH_3CO)_2O$) (30.63 g: 0.300 mol) was added dropwise to the reactor at a liquid temperature of 10°C to 20°C over 1 hour. After the completion of the dropwise addition, the reaction mixture was stirred at a liquid temperature of 20°C to 25°C for 1 hour, followed by stirring at a liquid temperature of 30°C to 35°C for 12 hours. The reaction mixture was cooled to no more than 10°C and, then, water (150.0 g) was dropped to the reactor to quench the reaction. Diethyl ether (250.0 g) was added to the reaction mixture for extraction and layer-separated into an organic layer and an aqueous layer. The organic layer thus prepared was washed with 3 wt% hydrochloric acid (250.0 g), 5 wt% sodium bicarbonate (250.0 g), and an aqueous 20 wt% sodium chloride solution (200.0 g) in this order. The solvent was removed from the washed organic layer at a reduced pressure, and the obtained crude product was purified by distillation at a reduced pressure to obtain 2-(1,5,5-trimethyl-2-cyclopentenyl)ethyl acetate (3) (36.51 g: 0.186 mol, yield 93.0%, purity 93.4%).

The following are various spectrum data of 2-(1,5,5-trimethyl-2-cyclopentenyl)ethyl acetate (3) thus prepared.

Nuclear magnetic resonance spectrum: $^1H$-NMR (500 MHz, $CDCl_3$): δ 0.88 (3H, s), 0.93 (3H, s), 0.94 (3H, s), 1.52-1.58 (1H, m), 1.66-1.71 (1H, m), 2.03 (1H, s), 2.11 (2H, t, J = 2.3 Hz), 4.07 (1H, ddd, J = 10.7, 9.6, 6.1 Hz), 4.18 (1H, ddd, J = 10.7, 9.6, 5.7 Hz), 5.54 (1H, dt, J = 6.2, 2.7 Hz), 5.61 (1H, dt, J = 5.9, 2.6 Hz) ppm. $^{13}C$-NMR (126 MHz, $CDCl_3$): δ 19.33, 21.04, 24.01, 24.61, 34.53,4,4.02, 46.77, 49.38, 62.71, 128.17, 138.68, 171.11 ppm.

Mass spectrum EI (70 eV): m/z 196 ($M^+$), 136, 121, 109, 93, 81, 80, 79, 77, 67, 55, 43, 29.

Infrared absorption spectrum (D-ATR): ν ($cm^{-1}$) 716, 961, 1031, 1053, 1235, 1387, 1452, 1743, 2843, 2872, 2967, 3051.

Comparative Example

The following Comparative example 1 describes a process for preparing ethyl (1,5,5-trimethyl-2-cyclopentenyl)acetate (2: R = Et) from the aforementioned 3,4,4-trimethyl-2-cyclopenten-1-ol (8) using a Johnson-Claisen rearrangement reaction described in Non-Patent Literature 3.

Comparative Example 1

Preparation of ethyl (1,5,5-trimethyl-2-cyclopentenyl)acetate (2: R = Et)

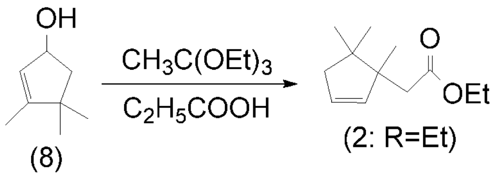


(8)

(2: R=Et)

Air in a reactor equipped with a stirrer, a condenser, a distillation tower, and a thermometer was purged with nitrogen. Then, to the reactor were added 3,4,4-trimethyl-2-cyclopenten-1-ol (8) (12.62 g: 0.100 mol, purity 94.9%), triethyl orthoacetate ($CH_3C(OEt)_3$) (81.12 g: 0.500 mol), and propionic acid ($C_2H_5COOH$) (0.74 g:0.010 mol), and the mixture was then stirred at a liquid temperature of 140°C to 145°C for 38 hours while distilling the refluxing ethanol off from a head of a fractional distillation tower. After the completion of the stirring, the reaction mixture was cooled to a liquid temperature of 20°C to 25°C. The excess triethyl orthoacetate was removed from the reaction mixture at a reduced pressure, and the crude product was then purified by silica gel column chromatography to obtain ethyl (1,5,5-trimethyl-2-cyclopentenyl)acetate (2: R = Et) (5.95 g: 0.030 mol, yield 30.3%).

The $^1$H-NMR Nuclear magnetic resonance spectrum and mass spectrum data of the ethyl (1,5,5-trimethyl-2-cyclopentenyl)acetate (2: R = Et) thus prepared were the same as those determined in Example 1-1.

The yield of ethyl (1,5,5-trimethyl-2-cyclopentenyl)acetate (2: R = Et) obtained in Comparative Example 1 was 30.3 % which was lower than the yield of 49.7% in Example 1-1, the yield of 47.8% in Example 1-2, the yield of 43.9% in Example 1-3, and the yield of 48.4% in Example 1-4.

## Claims

**1.** A process for preparing 2-(1,5,5-trimethyl-2-cyclopentenyl)ethyl acetate (3) of the following formula (3):

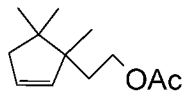

(3)

wherein Ac represents an acetyl group,
the process comprising:
subjecting a haloacetaldehyde alkyl 3,4,4-trimethyl-2-cyclopentenyl acetal compound of the following general formula (1):

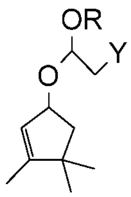

(1)

wherein R represents a linear or branched alkyl group having 1 to 4 carbon atoms, and Y represents a halogen atom,
to a dehydrohalogenation reaction in the presence of a base, followed by a rearrangement reaction to obtain a (1,5,5-trimethyl-2-cyclopentenyl)acetate compound of the following general formula (2):

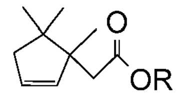

(2)

wherein R is as defined above, and
subjecting the alkoxycarbonylmethyl group (i.e., $-CH_2C(=O)OR$) of the (1,5,5-trimethyl-2-cyclopentenyl)acetate compound (2) to a multi-step conversion to be converted into a 2-acetoxyethyl group (i.e., $-CH_2CH_2OAc$) to obtain 2-(1,5,5-trimethyl-2-cyclopentenyl)ethyl acetate (3).

2. The process according to claim 1, wherein the multi-step conversion comprises a reduction reaction, followed by an acetylation reaction.

3. The process according to claim 1, wherein the multi-step conversion comprises a hydrolysis reaction, followed by a reduction reaction, and then an acetylation reaction.

## Patentansprüche

1. Ein Verfahren zur Herstellung von 2-(1,5,5-Trimethyl-2-cyclopentenyl)ethylacetat (3) der folgenden Formel (3):

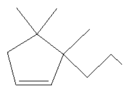

(3)

wobei Ac eine Acetylgruppe darstellt,
wobei das Verfahren umfasst:
Unterziehen einer Halogenacetaldehyd-alkyl-3,4,4-trimethyl-2-cyclopentenylacetal-Verbindung der folgenden allgemeinen Formel (1):

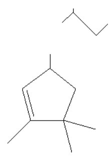

(1)

wobei R eine lineare oder verzweigte Alkylgruppe mit 1 bis 4 Kohlenstoffatomen darstellt und Y ein Halogenatom darstellt,
einer Dehydrohalogenierungsreaktion in Gegenwart einer Base, gefolgt von einer Umlagerungsreaktion, um eine (1,5,5-Trimethyl-2-cyclopentenyl)acetat-Verbindung der folgenden allgemeinen Formel (2) zu erhalten:

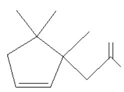

(2)

wobei R wie vorstehend definiert ist, und
Unterziehen der Alkoxycarbonylmethylgruppe (d.h. $-CH_2C(=O)OR$) der (1,5,5-Trimethyl-2-cyclopentenyl)acetat-Verbindung (2) einer mehrstufigen Umwandlung, um sie in eine 2-Acetoxyethylgruppe (d.h. $-CH_2CH_2OAc$) umzuwandeln, um 2-(1,5,5-Trimethyl-2-cyclopentenyl)ethylacetat (3) zu erhalten.

2. Das Verfahren nach Anspruch 1, wobei die mehrstufige Umwandlung eine Reduktionsreaktion, gefolgt von einer Acetylierungsreaktion, umfasst.

3. Das Verfahren nach Anspruch 1, wobei die mehrstufige Umwandlung eine Hydrolysereaktion, gefolgt von einer Reduktionsreaktion, und dann eine Acetylierungsreaktion umfasst.

## Revendications

1. Procédé de préparation d'acétate de 2-(1,5,5-triméthyl-2-cyclopentènyl)éthyle (3) de formule (3) suivante :

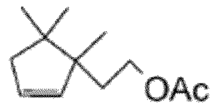

(3)

dans lequel Ac représente un groupe acétyle,
le procédé comprenant :
la soumission d'un composé haloacétaldéhyde alkyle 3,4,4-triméthyl-2-cyclopentènyl acétal de formule générale (1) suivante :

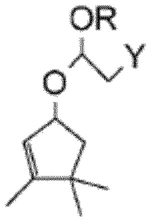

(1)

dans lequel R représente un groupe alkyle linéaire ou ramifié ayant 1 à 4 atomes de carbone, et Y représente un atome d'halogène,
à une réaction de déshydrohalogénation en présence d'une base, suivie d'une réaction de réarrangement pour obtenir un composé acétate de (1,5,5-triméthyl-2-cyclopentènyle) de formule générale (2) suivante :

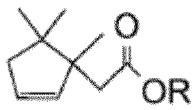

(2)

dans lequel R est tel que défini ci-dessus, et
la soumission du groupe alcoxycarbonylméthyle (c'est-à-dire $-CH_2C(=O)OR$) du composé acétate de (1,5,5-triméthyl-2-cyclopentènyle) (2) à une conversion en plusieurs étapes pour le convertir en un groupe 2-acétoxyéthyle (c'est-à-dire $-CH_2CH_2OAc$) pour obtenir l'acétate de 2-(1,5,5-triméthyl-2-cyclopentènyl)éthyle (3).

2. Procédé selon la revendication 1, dans lequel la conversion en plusieurs étapes comprend une réaction de réduction, suivie d'une réaction d'acétylation.

3. Procédé selon la revendication 1, dans lequel la conversion en plusieurs étapes comprend une réaction d'hydrolyse, suivie d'une réaction de réduction, puis d'une réaction d'acétylation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **R. RAMESH et al.** *J. Org. Chem.,* 2015, vol. 80, 7785-7789
- **J. G. MILLAR et al.** *Synlett,* 2010, vol. 15, 2319-2321
- **W. S. JOHNSON et al.** *J. Am. Chem. Soc.,* 1970, vol. 92 (92), 741-743